# EUROPEAN PATENT SPECIFICATION

(11) **EP 4 085 156 B1**
(45) Date of publication and mention of the grant of the patent: **25.10.2023**
(21) Application number: 20842231.1
(22) Date of filing: 28.12.2020
(51) Int. Cl.: C12Q 1/70

(54) **QUANTITATIVE PCR SCREENING OF INDUCIBLE PROPHAGE FROM BACTERIAL ISOLATES**
QUANTITATIVES PCR-SCREENING VON INDUZIERBAREN PROPHAGEN AUS BAKTERIENISOLATEN
CRIBLAGE PAR PCR QUANTITATIVE DE PROPHAGES INDUCTIBLES À PARTIR D'ISOLATS BACTÉRIENS

(30) Priority: 31.12.2019 US 201962955746 P
(43) Date of publication of application: 09.11.2022
(73) Proprietor: F. Hoffmann-La Roche AG, 4070 Basel (CH); Roche Diagnostics GmbH, 68305 Mannheim (DE)
(72) Inventor: CHIU, Sharon, Ho-Chen, Pleasanton, California 94588 (US); LIU, Xiaowen, Pleasanton, California 94588 (US); REHFUSS, Marc, Pleasanton, California 94588 (US); SUN, Jingtao, Pleasanton, California 94588 (US); YOUNGBLUT, Matthew, Pleasanton, California 94588 (US); ZHANG, Rui, Pleasanton, California 94588 (US)
(74) Representative: Hövelmann, Sascha Alexander
(86) International application number: PCT/EP2020/087902
(87) International publication number: WO 2021/136752

(56) References cited:
- US-A1- 2016 281 179
- US-A1- 2017 166 907
- ZHAO YANLIN ET AL: "ABSTRACT", APPLIED AND ENVIRONMENTAL MICROBIOLOGY, vol. 76, no. 2, 30 November 2009 (2009-11-30), pages 589-595, XP055793532, US ISSN: 0099-2240, DOI: 10.1128/AEM.01450-09
- CHEN F ET AL: "Induction of multiple prophages from a marine bacterium: a genomic approach", APPLIED AND ENVIRONMENTAL MICROBIOLOGY, AMERICAN SOCIETY FOR MICROBIOLOGY, US, vol. 72, no. 7, 1 July 2006 (2006-07-01), pages 4995-5001, XP002551082, ISSN: 0099-2240, DOI: 10.1128/AEM.00056-06 cited in the application

## Description

### BACKGROUND OF THE INVENTION

### Field of the invention

The invention relates to methods and compositions for performing quantitative polymerase chain reaction to screen for previously undiscovered inducible prophages from various bacterial strains.

### Description of the Related Art

Bacteriophages are abundant in the biosphere (Clokie, et al., 2011), and 70% of sequenced bacterial genomes contain prophage-like DNA sequences (Chen, et al., 2006). As a new assay reagent for rapid diagnostic test for bacterial infection) such as the Smarticles technology described in U.S Patent No. 9,388,453 relies on this natural abundance of prophages in nature to isolate novel phages specific to bacterial species, and convert the native phage to Smarticles as a delivery vehicle for reporter DNA delivery. However, not all prophages-like DNA sequences are viable prophages that are functional, or inducible under standard induction conditions.

Conventional functional prophage identification relies on the lytic property of prophage under induction. Genome-based identification of prophage by next generation sequencing has gained popularity recently (Zhou, et al., 2011, Hertel et al., 2015). Bioinformatics based genome identification, for example, identifies putative prophage by annotation of prophage sequences within bacterial genomes and plasmids (http://phaster.ca/) (Arndt, et al., 2016). Yet, distinguishing viable prophages from nonviable sequences can be a challenge, as non-active prophages caused by genetic mutations can only be determined by functional data (Zhao, et al., 2010).

Therefore, there is a need in the art to develop a quantitative polymerase chain reaction (qPCR) method to quickly identify viable and inducible prophages from any given bacterial species. Briefly, the method relies on the DNA copy number differences of prophage DNA upon phage induction. Upon induction, viable and inducible prophage undergoes lytic cycle, propagation, and the phages are released into the bacterial cell culture medium, while non-inducible and nonviable phage DNA remains inactive, thus maintains the same DNA copy number as the bacterial chromosome DNA. Consensus phage terminase DNA sequences are used for primer design, and qPCR are performed on induced and non-induced bacterial lysate. Inducible and viable prophage is defined with a DeltaCt (ΔCt) cutoff ΔCt > 2. By combining with plaquing result, this method can rapidly identify inducible, and broad host-range prophages for creation of NRTPs

US 2017/166907 A1 teaches a method of identifying viable prophages by inducing a lytic cycle and determining electron microscopically the production of viral particles.

US 2016/281179 A1 teaches the use of a packaging plasmid that is derived from the P1 or ϕ80 bacteriophage but having a non-functional packaging initiation site and thus inherently comprises a terminal small unit gene and a terminal large unit gene in producing a non-replicative transduction particle.

### SUMMARY OF THE INVENTION

The invention is set out in the appended set of claims.

The present invention relates to methods and compositions for using qPCR to determine the presence of inducible bacteriophage (existing as prophage) for the purpose of generating non-replicative transduction particles (e.g. Smarticles). Therefore in one aspect, the present invention relates to a method of identifying the presence of a viable and inducible prophage from a bacterial species that is not known to contain said viable and inducible prophage, said method comprising: providing a pair of oligonucleotides to be used as a forward primer and a reverse primer to amplify a segment of a bacteriophage terminase large unit gene; performing two quantitative polymerase chain reaction (qPCR) experiments with said forward and reverse primers, wherein one experiment is performed on a culture of said bacterial species that is induced to allow the prophage to undergo the lytic cycle, and the other experiment is performed on a culture of said bacterial species that is not induced; comparing the cycle threshold (Ct) values of the two qPCR experiments, wherein a difference of Ct value (ΔCt) between the induced culture and the non-induced culture is greater than two is indicative of the presence of viable and inducible prophage from said bacterial species, and wherein a ΔCt between the induced culture and the non-induced culture is less than two is indicative of the absence of viable and inducible prophage from said bacterial species.

In one embodiment, the method of the present invention further comprises a step of determining the melting temperature (Tm) of the amplified segment wherein a Tm that is greater than 81°C and less than 89°C is indicative of the presence of viable and inducible prophage from said bacterial species. In another embodiment, the nucleotide sequences of the forward primer and the reverse primer are derived from the sequences of bacteriophage terminase large unit genes that have been identified from said bacterial species. In yet another embodiment, the bacterial species is *Klebsiella pneumonia* (Kpn). In one embodiment, the nucleotide sequence of the Kpn forward primer is selected from SEQ ID NO: 1-12 and the nucleotide sequence of the Kpn reverse primer is selected from SEQ ID NO: 13-24. In another embodiment, the bacterial species is *Enterobacter cloacae* (Ecl) and the nucleotide sequence of the Ecl forward primer is selected from SEQ ID NO: 25-34 and the nucleotide sequence of the Ecl reverse primer is selected from SEQ ID NO: 35-44. In yet another embodiment, the bacterial species is *Escherichia coli* (Eco) and the nucleotide sequence of the Eco forward primer is selected from SEQ ID NO: 45-62 and the nucleotide sequence of the Eco reverse primer is selected from SEQ ID NO: 63-80. In still another embodiment, the bacterial species is *Pseudomonas aeruginosa* (Pae) and the nucleotide sequence of the Pae forward primer is selected from SEQ ID NOs: 82, 84, 86 or 88 and the nucleotide sequence of the Pae reverse primer is selected from SEQ ID NOs: 83, 85, 87 or 89. The present invention also relates to a packaging plasmid comprising a terminase small unit gene and a terminase large unit gene derived from an induced prophage, wherein the induced prophage is derived from *Klebsiella pneumonia* (Kpn) and comprises a nucleotide sequence of pZX023 (SEQ ID NO: 81). Also disclosed is that the induced prophage is derived from *Enterobacter cloacae* (Ecl), that the induced prophage is derived from *Escherichia coli* (Eco) and that the induced prophage is derived from *Pseudomonas aeruginosa* (Pae). In yet another aspect, the present invention relates to a non-replicative transduction particle that comprises the packaging plasmid comprising a terminase small unit gene and a terminase large unit gene derived from an induced prophage, wherein the induced prophage is derived from *Klebsiella pneumonia* (Kpn) and comprises a nucleotide sequence of pZX023 (SEQ ID NO: 81).

The present invention also relates to a method of identifying an inducible prophage from a bacteria species for creation of a functional non-replicative transcription particle (NRTP) that is characterized by delivering into a cell a reporter molecule comprising a detectable reporter gene and expressing the detectable reporter gene in the cell, said method comprising: providing a pair of oligonucleotides to be used as a forward primer and a reverse primer to amplify a segment of a bacteriophage terminase large unit gene or a bacteriophage tail fiber protein gene; performing two quantitative polymerase chain reaction (qPCR) experiments with said forward and reverse primers, wherein one experiment is performed on a culture of said bacterial species that is induced to allow the prophage to undergo the lytic cycle, and the other experiment is performed on a culture of said bacterial species that is not induced; comparing the cycle threshold (Ct) values of the two qPCR experiments, wherein a difference of Ct value (ΔCt) between the induced culture and the non-induced culture is greater than two is indicative of the ability of the bacteriophage to generate the functional NRTP, and wherein a ΔCt value between the induced culture and the non-induced culture is less than two is indicative of the inability of the bacteriophage to generate the functional NRTP. In one embodiment, the bacteria species is from the Enterobacteriales order. In one embodiment, the bacteria species is *Citrobacter freundii* (Cfi) and the nucleotide sequence of the forward primer is selected from SEQ ID NO: 90 or 92 and the nucleotide sequence of the reverse primer is selected from SEQ ID NO: 91 or 93. In another embodiment, the bacteria species is *Serratia marcescens* (Sms) and the nucleotide sequence of the forward primer is selected from SEQ ID NO: 94 or 96 and the nucleotide sequence of the reverse primer is selected from SEQ ID NO: 95 or 97.

### BRIEF DESCRIPTION OF THE DRAWINGS

FIG. 1 shows growth curves of a qPCR experiment using Kpn clade 11-12 as primers before induction and after induction of an inducible prophage (left) and a non-inducible prophage (right).
FIG. 2 shows growth curves of a qPCR experiment using Eco clade 11-12 as primers before induction and after induction of an inducible prophage (left) and a non-inducible prophage (right).
FIG. 3 shows growth curves of a qPCR experiment using Ecl clade 8-9 as primers before induction and after induction of an inducible prophage (left) and a non-inducible prophage (right).
FIG. 4 shows a plasmid map of pZX023.
FIG. 5 shows the growth curves of a qPCR experiment with strain Pae0060-F10 phage using the primers and experimental conditions as described in Example 4.
FIG. 6 shows the growth curves of a qPCR experiment with strain Pae0065-F10 phage using the primers and experimental conditions as described in Example 4.
FIG. 7 shows the growth curves of a qPCR experiment with strain Pae0042-phi297 phage using the primers and experimental conditions as described in Example 4.
FIG. 8 shows the growth curves of a qPCR experiment with strain Pae0057-phi3 phage using the primers and experimental conditions as described in Example 4.
FIG. 9 shows the growth curves of a qPCR experiment with strain Cfi16709-P1 phage using primers and experimental conditions as described in Example 5.
FIG. 10 shows the growth curves of a qPCR experiment with strain Cfi 167 10- phiKO2 phage using primers and experimental conditions as described in Example 5.
FIG. 11 shows the growth curves of a qPCR experiment with strain Sms16017- SEN34 phage using the primers and experimental conditions as described in Example 5.
FIG. 12 shows the growth curves of a qPCR experiment with strain Sms16926-Psp3 phage using the primers and experimental conditions as described in Example 5.

### DETAILED DESCRIPTION OF THE INVENTION

### I. Definitions

Terms used in the claims and specification are defined as set forth below unless otherwise specified.

As used herein, "reporter nucleic acid molecule" refers to a nucleotide sequence comprising a DNA or RNA molecule. The reporter nucleic acid molecule can be naturally occurring or an artificial or synthetic molecule. In some embodiments, the reporter nucleic acid molecule is exogenous to a host cell and can be introduced into a host cell as part of an exogenous nucleic acid molecule, such as a plasmid or vector. In certain embodiments, the reporter nucleic acid molecule can be complementary to a target gene in a cell. In other embodiments, the reporter nucleic acid molecule comprises a reporter gene encoding a reporter molecule (e.g., reporter enzyme, protein). In some embodiments, the reporter nucleic acid molecule is referred to as a "reporter construct" or "nucleic acid reporter construct."

A "reporter molecule" or "reporter" refers to a molecule (e.g., nucleic acid or protein) that confers onto an organism a detectable or selectable phenotype. The detectable phenotype can be colorimetric, fluorescent or luminescent, for example. Reporter molecules can be expressed from reporter genes encoding enzymes mediating luminescence reactions (LuxA, LuxB, LuxAB, Luc, Ruc, nLuc), genes encoding enzymes mediating colorimetric reactions (LacZ, HRP), genes encoding fluorescent proteins (GFP, eGFP, YFP, RFP, CFP, BFP, mCherry, near-infrared fluorescent proteins), nucleic acid molecules encoding affinity peptides (His-tag, 3X-FLAG), and genes encoding selectable markers (e.g. ampC, tet(M), zeoR, hph, CAT, erm). The reporter molecule can be used as a marker for successful uptake of a nucleic acid molecule or exogenous sequence (plasmid) into a cell. The reporter molecule can also be used to indicate the presence of a target gene, target nucleic acid molecule, target intracellular molecule, or a cell, as described herein. Alternatively, the reporter molecule can be a nucleic acid, such as an aptamer or ribozyme.

In some aspects of the invention, the reporter nucleic acid molecule is operatively linked to a promoter. In other aspects of the invention, the promoter can be chosen or designed to contribute to the reactivity and cross-reactivity of the reporter system based on the activity of the promoter in specific cells (*e.g.,* specific species) and not in others. In certain aspects, the reporter nucleic acid molecule comprises an origin of replication. In other aspects, the choice of origin of replication can similarly contribute to reactivity and cross-reactivity of the reporter system, when replication of the reporter nucleic acid molecule within the target cell contributes to or is required for reporter signal production based on the activity of the origin of replication in specific cells (*e.g.,* specific species) and not in others. In some embodiments, the reporter nucleic acid molecule forms a replicon capable of being packaged as concatameric DNA into a progeny virus during virus replication.

As used herein, a "target transcript" refers to a portion of a nucleotide sequence of a DNA sequence or an mRNA molecule that is naturally formed by a target cell including that formed during the transcription of a target gene and mRNA that is a product of RNA processing of a primary transcription product. The target transcript can also be referred to as a cellular transcript or naturally occurring transcript.

As used herein, the term "transcript" refers to a length of nucleotide sequence (DNA or RNA) transcribed from a DNA or RNA template sequence or gene. The transcript can be a cDNA sequence transcribed from an RNA template or an mRNA sequence transcribed from a DNA template. The transcript can be protein coding or non-coding. The transcript can also be transcribed from an engineered nucleic acid construct.

A transcript derived from a reporter nucleic acid molecule can be referred to as a "reporter transcript." The reporter transcript can include a reporter sequence and a cis-repressing sequence. The reporter transcript can have sequences that form regions of complementarity, such that the transcript includes two regions that form a duplex (*e.g.,* an intermolecular duplex region). One region can be referred to as a "cis-repressing sequence" and has complementarity to a portion or all of a target transcript and/or a reporter sequence. A second region of the transcript is called a "reporter sequence" and can have complementarity to the cis-repressing sequence. Complementarity can be full complementarity or substantial complementarity. The presence and/or binding of the cis-repressing sequence with the reporter sequence can form a conformation in the reporter transcript, which can block further expression of the reporter molecule. The reporter transcript can form secondary structures, such as a hairpin structure, such that regions within the reporter transcript that are complementary to each other can hybridize to each other.

"Introducing into a cell," when referring to a nucleic acid molecule or exogenous sequence (*e.g.,* plasmid, vector, construct), means facilitating uptake or absorption into the cell, as is understood by those skilled in the art. Absorption or uptake of nucleic acid constructs or transcripts can occur through unaided diffusive or active cellular processes, or by auxiliary agents or devices including via the use of bacteriophage, virus, and transduction particles. The meaning of this term is not limited to cells *in vitro;* a nucleic acid molecule may also be "introduced into a cell," wherein the cell is part of a living organism. In such instance, introduction into the cell will include the delivery to the organism. For example, for *in vivo* delivery, nucleic acid molecules, constructs or vectors of the invention can be injected into a tissue site or administered systemically. *In vitro* introduction into a cell includes methods known in the art, such as electroporation and lipofection. Further approaches are described herein or known in the art.

A "transduction particle" refers to a virus capable of delivering a non-viral nucleic acid molecule into a cell. The virus can be a bacteriophage, adenovirus, etc.

A "non-replicative transduction particle" or "NRTP" refers to a virus capable of delivering a non-viral nucleic acid molecule into a cell, but is incapable of packaging its own replicated viral genome into the transduction particle. The virus can be a bacteriophage, adenovirus, *etc.*

A "plasmid" is a small DNA molecule that is physically separate from, and can replicate independently of, chromosomal DNA within a cell. Most commonly found as small circular, double-stranded DNA molecules in bacteria, plasmids are sometimes present in archaea and eukaryotic organisms. Plasmids are considered replicons, capable of replicating autonomously within a suitable host.

A "vector" is a nucleic acid molecule used as a vehicle to artificially carry foreign genetic material into another cell, where it can be replicated and/or expressed.

A "virus" is a small infectious agent that replicates only inside the living cells of other organisms. Virus particles (known as virions) include two or three parts: i) the genetic material made from either DNA or RNA molecules that carry genetic information; ii) a protein coat that protects these genes; and in some cases, iii) an envelope of lipids that 9388

As used herein, the term "complement" refers to a non-disrupted sequence that is in the presence of an identical sequence that has been disrupted, or to the relationship of the non-disrupted sequence to the disrupted sequence. In one embodiment, the complement comprises a gene encoded on a polynucleotide in a cell that is functional and capable of expression, and expresses a protein with the same function as a disrupted gene on a bacteriophage prior to disruption. In some embodiments, the complement gene has greater than 80%, 85%, 90%, 91%, 92%, 93%, 94%, 95%, 96%, 97%, 98%, or 99% sequence identity to the disrupted bacteriophage gene prior to disruption, i.e., the native bacteriophage gene. In some embodiments, the complement gene is identical to the disrupted bacteriophage gene prior to disruption, i.e., the native bacteriophage gene. In some embodiments, the complement gene comprises a polynucleotide sequence that has been deleted from the bacteriophage. In some embodiments, the complement gene refers to a gene encoding packaging machinery of a bacteriophage on a plasmid, where the same gene has been disrupted in a bacteriophage. Thus, the plasmid is required to be in the presence of a bacteriophage with a mutated packaging machinery gene to provide the necessary packaging machinery necessary for packaging a polynucleotide into a transduction particle.

As used herein, the term "packaging-related enzymatic activity" refers to one or more polypeptides crucial for the interaction with a packaging initiation site sequence to package a polynucleotide into a transduction particle. In some embodiments, a pair of terminase genes is required for such an interaction, wherein each terminase encodes a packaging-related enzymatic activity. In some embodiments, the enzymatic activity is encoded by a terS and/or terL gene from bacteriophages that were discovered in the present invention. In these embodiments, each of the pair of terminase genes express a packaging-related enzymatic activity, and a functional version of both are required for packaging of a polynucleotide with the packaging initiation site. In some embodiments, disruption of one of the genes of a plurality of genes associated with a packaging-related enzymatic activity eliminates the packaging-related enzymatic activity. In some embodiments, both of the pair of terminase genes are disrupted on the bacteriophage genome, thus disrupting the entire set of packaging-related enzymatic activity encoding genes on the bacteriophage.

The term "ameliorating" refers to any therapeutically beneficial result in the treatment of a disease state, *e.g.,* a disease state, including prophylaxis, lessening in the severity or progression, remission, or cure thereof.

The term "in situ" refers to processes that occur in a living cell growing separate from a living organism, *e.g.,* growing in tissue culture.

The term "in vivo" refers to processes that occur in a living organism.

The term "mammal" as used herein includes both humans and non-humans and include but is not limited to humans, non-human primates, canines, felines, murines, bovines, equines, and porcines.

"G," "C," "A" and "U" each generally stand for a nucleotide that contains guanine, cytosine, adenine, and uracil as a base, respectively. "T" and "dT" are used interchangeably herein and refer to a deoxyribonucleotide wherein the nucleobase is thymine, *e.g.,* deoxyribothymine. However, it will be understood that the term "ribonucleotide" or "nucleotide" or "deoxyribonucleotide" can also refer to a modified nucleotide, as further detailed below, or a surrogate replacement moiety. The skilled person is well aware that guanine, cytosine, adenine, and uracil may be replaced by other moieties without substantially altering the base pairing properties of an oligonucleotide comprising a nucleotide bearing such replacement moiety. For example, without limitation, a nucleotide comprising inosine as its base may base pair with nucleotides containing adenine, cytosine, or uracil. Hence, nucleotides containing uracil, guanine, or adenine may be replaced in the nucleotide sequences of the invention by a nucleotide containing, for example, inosine. Sequences comprising such replacement moieties are embodiments of the invention.

As used herein, the term "complementary," when used to describe a first nucleotide sequence in relation to a second nucleotide sequence, refers to the ability of an oligonucleotide or polynucleotide comprising the first nucleotide sequence to hybridize and form a duplex structure under certain conditions with an oligonucleotide or polynucleotide comprising the second nucleotide sequence, as will be understood by the skilled person. Complementary sequences are also described as binding to each other and characterized by binding affinities. For example, a first nucleotide sequence can be described as complementary to a second nucleotide sequence when the two sequences hybridize (*e.g.,* anneal) under stringent hybridization conditions. Hybridization conditions include temperature, ionic strength, pH, and organic solvent concentration for the annealing and/or washing steps. The term stringent hybridization conditions refers to conditions under which a first nucleotide sequence will hybridize preferentially to its target sequence, e.g., a second nucleotide sequence, and to a lesser extent to, or not at all to, other sequences. Stringent hybridization conditions are sequence dependent, and are different under different environmental parameters. Generally, stringent hybridization conditions are selected to be about 5°C lower than the thermal melting point (Tₘ) for the nucleotide sequence at a defined ionic strength and pH. The Tₘ is the temperature (under defined ionic strength and pH) at which 50% of the first nucleotide sequences hybridize to a perfectly matched target sequence. An extensive guide to the hybridization of nucleic acids is found in, *e.g.,* Tijssen (1993) Laboratory Techniques in Biochemistry and Molecular Biology--Hybridization with Nucleic Acid Probes part I, chap. 2, "Overview of principles of hybridization and the strategy of nucleic acid probe assays," Elsevier, N.Y. ("Tijssen"). Other conditions, such as physiologically relevant conditions as may be encountered inside an organism, can apply. The skilled person will be able to determine the set of conditions most appropriate for a test of complementarity of two sequences in accordance with the ultimate application of the hybridized nucleotides.

This includes base-pairing of the oligonucleotide or polynucleotide comprising the first nucleotide sequence to the oligonucleotide or polynucleotide comprising the second nucleotide sequence over the entire length of the first and second nucleotide sequence. Such sequences can be referred to as "fully complementary" with respect to each other herein. However, where a first sequence is referred to as "substantially complementary" with respect to a second sequence herein, the two sequences can be fully complementary, or they may form one or more, but generally not more than 4, 3 or 2 mismatched base pairs upon hybridization, while retaining the ability to hybridize under the conditions most relevant to their ultimate application. However, where two oligonucleotides are designed to form, upon hybridization, one or more single stranded overhangs, such overhangs shall not be regarded as mismatches with regard to the determination of complementarity. For example, a dsRNA comprising one oligonucleotide 21 nucleotides in length and another oligonucleotide 23 nucleotides in length, wherein the longer oligonucleotide comprises a sequence of 21 nucleotides that is fully complementary to the shorter oligonucleotide, may yet be referred to as "fully complementary" for the purposes described herein.

"Complementary" sequences, as used herein, may also include, or be formed entirely from, non-Watson-Crick base pairs and/or base pairs formed from non-natural and modified nucleotides, provided the above requirements with respect to their ability to hybridize are fulfilled. Such non-Watson-Crick base pairs includes, but not limited to, G:U Wobble or Hoogstein base pairing.

The terms "complementary," "fully complementary" and "substantially complementary" herein may be used with respect to the base matching between two strands of a dsRNA, or between the antisense strand of a dsRNA and a target sequence, between complementary strands of a single stranded RNA sequence or a single stranded DNA sequence, as will be understood from the context of their use.

As used herein, a "duplex structure" comprises two anti-parallel and substantially complementary nucleic acid sequences. Complementary sequences in a nucleic acid construct, between two transcripts, between two regions within a transcript, or between a transcript and a target sequence can form a "duplex structure." In general, the majority of nucleotides of each strand are ribonucleotides, but as described in detail herein, each or both strands can also include at least one non-ribonucleotide, *e.g.,* a deoxyribonucleotide and/or a modified nucleotide. The two strands forming the duplex structure may be different portions of one larger RNA molecule, or they may be separate RNA molecules. Where the two strands are part of one larger molecule, and therefore are connected by an uninterrupted chain of nucleotides between the 3'-end of one strand and the 5'-end of the respective other strand forming the duplex structure, the connecting RNA chain is referred to as a "hairpin loop." Where the two strands are connected covalently by means other than an uninterrupted chain of nucleotides between the 3'-end of one strand and the 5'-end of the respective other strand forming the duplex structure, the connecting structure is referred to as a "linker." The RNA strands may have the same or a different number of nucleotides. The maximum number of base pairs is the number of nucleotides in the shortest strand of the duplex minus any overhangs that are present in the duplex. Generally, the duplex structure is between 15 and 30 or between 25 and 30, or between 18 and 25, or between 19 and 24, or between 19 and 21, or 19, 20, or 21 base pairs in length. In one embodiment the duplex is 19 base pairs in length. In another embodiment the duplex is 21 base pairs in length. When two different siRNAs are used in combination, the duplex lengths can be identical or can differ. As used herein, the term "region of complementarity" refers to the region on the antisense strand that is substantially complementary to a sequence, for example a target sequence, as defined herein. Where the region of complementarity is not fully complementary to the target sequence, the mismatches are most tolerated in the terminal regions and, if present, are generally in a terminal region or regions, *e.g.,* within 6, 5, 4, 3, or 2 nucleotides of the 5' and/or 3' terminus. The term "percent identity," in the context of two or more nucleic acid or polypeptide sequences, refer to two or more sequences or subsequences that have a specified percentage of nucleotides or amino acid residues that are the same, when compared and aligned for maximum correspondence, as measured using one of the sequence comparison algorithms described below (*e.g.,* BLASTP and BLASTN or other algorithms available to persons of skill) or by visual inspection. Depending on the application, the percent "identity" can exist over a region of the sequence being compared, *e.g.,* over a functional domain, or, alternatively, exist over the full length of the two sequences to be compared.

For sequence comparison, typically one sequence acts as a reference sequence to which test sequences are compared. When using a sequence comparison algorithm, test and reference sequences are input into a computer, subsequence coordinates are designated, if necessary, and sequence algorithm program parameters are designated. The sequence comparison algorithm then calculates the percent sequence identity for the test sequence(s) relative to the reference sequence, based on the designated program parameters.

Optimal alignment of sequences for comparison can be conducted, *e.g.,* by the local homology algorithm of Smith & Waterman, Adv. Appl. Math. 2:482 (1981), by the homology alignment algorithm of Needleman & Wunsch, J. Mol. Biol. 48:443 (1970), by the search for similarity method of Pearson & Lipman, Proc. Nat'l. Acad. Sci. USA 85:2444 (1988), by computerized implementations of these algorithms (GAP, BESTFIT, FASTA, and TFASTA in the Wisconsin Genetics Software Package, Genetics Computer Group, 575 Science Dr., Madison, Wis.), or by visual inspection (see generally Ausubel *et al.,* infra).

One example of an algorithm that is suitable for determining percent sequence identity and sequence similarity is the BLAST algorithm, which is described in Altschul et al., J. Mol. Biol. 215:403-410 (1990). Software for performing BLAST analyses is publicly available through the National Center for Biotechnology Information (www.ncbi.nlm.nih.gov/).

The disclosed methods may include performing at least one cycling step that includes amplifying one or more portions of the nucleic acid molecule gene target from a sample using one or more pairs of primers. A "sample" or "biological sample" as used herein refers to a sample that includes but is not limited to whole blood, plasma, serum, red blood cell fraction, saliva, cerebrospinal fluid, semen, urine, seat, bile, lymph, sputum, lavage fluid, or a combination thereof. "Primer(s)" as used herein refer to oligonucleotide primers that specifically anneal to the target bacterial gene, and initiate DNA synthesis therefrom under appropriate conditions producing the respective amplification products. Each of the discussed primers anneals to a target within or adjacent to the respective target nucleic acid molecule such that at least a portion of each amplification product contains nucleic acid sequence corresponding to the target. The one or more amplification products are produced provided that one or more of the target bacterial gene nucleic acid is present in the sample, thus the presence of the one or more of target bacterial gene amplification products is indicative of the presence of that bacterial strain in the sample. The amplification product should contain the nucleic acid sequences that are complementary to one or more detectable probes for target bacterial gene. "Probe(s)" as used herein refer to oligonucleotide probes that specifically anneal to nucleic acid sequence encoding the target bacterial gene. Each cycling step includes an amplification step, a hybridization step, and a detection step, in which the sample is contacted with the one or more detectable probes for detection of the presence or absence of the bacterial strain in the sample. As used herein, the term "amplifying" refers to the process of synthesizing nucleic acid molecules that are complementary to one or both strands of a template nucleic acid molecule. Amplifying a nucleic acid molecule typically includes denaturing the template nucleic acid, annealing primers to the template nucleic acid at a temperature that is below the melting temperatures of the primers, and enzymatically elongating from the primers to generate an amplification product. Amplification typically requires the presence of deoxyribonucleoside triphosphates, a DNA polymerase enzyme (e.g., Platinum^{®} Taq) and an appropriate buffer and/or co-factors for optimal activity of the polymerase enzyme (e.g., MgCl2 and/or KCl). The term "primer" as used herein is known to those skilled in the art and refers to oligomeric compounds, primarily to oligonucleotides but also to modified oligonucleotides that are able to "prime" DNA synthesis by a template-dependent DNA polymerase, i.e., the 3'-end of the, e.g., oligonucleotide provides a free 3'-OH group whereto further "nucleotides" may be attached by a template-dependent DNA polymerase establishing 3' to 5' phosphodiester linkage whereby deoxynucleoside triphosphates are used and whereby pyrophosphate is released. Therefore, there is - except possibly for the intended function - no fundamental difference between a "primer", an "oligonucleotide", or a "probe".

The term "hybridizing" refers to the annealing of one or more probes to an amplification product. Hybridization conditions typically include a temperature that is below the melting temperature of the probes but that avoids non-specific hybridization of the probes.

The term "5' to 3' nuclease activity" refers to an activity of a nucleic acid polymerase, typically associated with the nucleic acid strand synthesis, whereby nucleotides are removed from the 5' end of nucleic acid strand.

The term "thermostable polymerase" refers to a polymerase enzyme that is heat stable, i.e., the enzyme catalyzes the formation of primer extension products complementary to a template and does not irreversibly denature when subjected to the elevated temperatures for the time necessary to effect denaturation of double-stranded template nucleic acids. Generally, the synthesis is initiated at the 3' end of each primer and proceeds in the 5' to 3' direction along the template strand. Thermostable polymerases have been isolated from *Thermus flavus, T. ruber, T. thermophilus, T. aquaticus, T. lacteus, T. rubens, Bacillus stearothermophilus, and Methanothermus fervidus.* Nonetheless, polymerases that are not thermostable also can be employed in PCR assays provided the enzyme is replenished.

The term "complement thereof' refers to nucleic acid that is both the same length as, and exactly complementary to, a given nucleic acid.

The term "extension" or "elongation" when used with respect to nucleic acids refers to when additional nucleotides (or other analogous molecules) are incorporated into the nucleic acids. For example, a nucleic acid is optionally extended by a nucleotide incorporating biocatalyst, such as a polymerase that typically adds nucleotides at the 3' terminal end of a nucleic acid.

A "modified nucleotide" in the context of an oligonucleotide refers to an alteration in which at least one nucleotide of the oligonucleotide sequence is replaced by a different nucleotide that provides a desired property to the oligonucleotide. Exemplary modified nucleotides that can be substituted in the oligonucleotides described herein include, e.g., a C5-methyl-dC, a C5-ethyl-dC, a C5-methyl-dU, a C5-ethyl-dU, a 2,6-diaminopurine, a C5-propynyl-dC, a C5-propynyl-dU, a C7-propynyl-dA, a C7-propynyl-dG, a C5-propargylamino-dC, a C5-propargylamino-dU, a C7-propargylamino-dA, a C7-propargylamino-dG, a 7-deaza-2-deoxyxanthosine, a pyrazolopyrimidine analog, a pseudo-dU, a nitro pyrrole, a nitro indole, 2'-0-methyl Ribo-U, 2'-0-methyl Ribo-C, an N4-ethyl-dC, an N6-methyl-dA, and the like. Many other modified nucleotides that can be substituted in the oligonucleotides are referred to herein or are otherwise known in the art. In certain embodiments, modified nucleotide substitutions modify melting temperatures (Tm) of the oligonucleotides relative to the melting temperatures of corresponding unmodified oligonucleotides. To further illustrate, certain modified nucleotide substitutions can reduce non-specific nucleic acid amplification (e.g., minimize primer dimer formation or the like), increase the yield of an intended target amplicon, and/or the like in some embodiments. Examples of these types of nucleic acid modifications are described in, e.g., U.S. Pat. No. 6,001,611.

The data from a qPCR experiment can be depicted as growth curves in which the number of cycles required for a detectable signal (such as fluorescence intensity from fluorescent dyes) to cross background (threshold) level, which is commonly referred as the Cycle threshold or "Ct value".

The term "sufficient amount" means an amount sufficient to produce a desired effect, *e.g.,* an amount sufficient to produce a detectable signal from a cell.

The term "therapeutically effective amount" is an amount that is effective to ameliorate a symptom of a disease. A therapeutically effective amount can be a "prophylactically effective amount" as prophylaxis can be considered therapy.

It must be noted that, as used in the specification and the appended claims, the singular forms "a," "an" and "the" include plural referents unless the context clearly dictates otherwise.

### II. Lysogenic and Lytic Cycle of Viruses

Viruses undergo lysogenic and lytic cycles in a host cell. If the lysogenic cycle is adopted, the phage chromosome can be integrated into the bacterial chromosome, or it can establish itself as a stable plasmid in the host, where it can remain dormant for long periods of time. If the lysogen is induced, the phage genome is excised from the bacterial chromosome and initiates the lytic cycle, which culminates in lysis of the cell and the release of phage particles. The lytic cycle leads to the production of new phage particles which are released by lysis of the host.

In addition, virus-based reporter assays, such as phage-based reporters, can suffer from limited reactivity (*i.e.,* analytical inclusivity) due to limits in the phage host range caused by host-based and prophage-derived phage resistance mechanisms. These resistance mechanisms target native phage nucleic acid that can result in the degradation or otherwise inhibition of the phage DNA and functions. Such resistance mechanisms include restriction systems that cleave phage DNA and CRISPR systems that inhibit phage-derived transcripts.

Both lytic activity and phage resistance can be inhibitory to assays based on reporter phages. Lytic activity can inhibit signal by destroying or otherwise inhibiting the cell in its ability to generate a detectable signal and thus affecting limits of detection by reducing the amount of detectable signal or preventing the generation of a detectable signal. Phage resistance mechanisms can limit the host range of the phage and limit the inclusivity of the phage-based reporter, similarly affecting limits of detection by reducing the amount of detectable signal or preventing the generation of a detectable signal. Both lytic activity and phage resistance caused by the incorporation of phage DNA in a reporter phage can lead to false-negative results in assays that incorporate these phage reporters.

### III. Methods for Producing Non-Replicative Transduction Particles (NRTP)

### Disruption/Complementation-Based Methods For Producing Non-Replicative Transduction Particles

Disclosed herein are non-replicative transduction particle packaging systems, referred herein also as Smarticles systems, based on disruption of a component of the genome of a virus that is recognized by the viral packaging machinery as the element from which genomic packaging is initiated during viral production. In an embodiment, this disruption disrupts a packaging initiation site from a bacteriophage, and also disrupts a terminase function. Examples of the disrupted elements include the pac-site sequence of pac-type bacteriophages and the cos-site sequence of cos-type bacteriophages. When the packaging initiation site sequence within the phage is disrupted, the phage cannot produce functional terminases. In an example, the pac-site is encoded within a pacA gene sequence, and terminase functions require both a functional PacA and PacB. Plasmid DNA is packaged into a phage capsid by complementing said disrupted terminases and including a recognizable packaging initiation site on the plasmid DNA.

Packaging initiation sites are often found within coding regions of genes that are essential to virus production. A region of the bacteriophage genome can be disrupted by an insertion, replacement, deletion, or mutation that disrupts the packaging initiation site. Examples of disruptions that accomplish this include, but are not limited to, an allelic exchange event that replaces a sequence on the bacteriophage genome that contains the packaging initiation site sequence with another sequence such as that of an antibiotic resistance gene, or the complete deletion of the small and large terminase genes. In an example employing the terminase genes *pacA* and *pacB, pacA* can be disrupted in a manner that causes polar effects that also disrupt pacB expression and/or overall terminase function mediated by PacA and PacB. Other examples can include the disruption of terminase genes and can also include *terS* and *terL* genes from bacteriophages discovered in the present invention

In one example, a cell's genome is lysogenized with a viral genome where the packaging initiation site has been disrupted. The cell can be Gram-negative or Gram-positive. A complementing plasmid (or reporter nucleic acid molecule) is introduced into the cell, and the plasmid DNA includes at least the gene that has been disrupted in the bacteriophage, as well as the packaging initiation site sequence, and optionally additional bacteriophage genes and a reporter gene, which can encode a detectable and/or a selectable marker. The plasmid can be constructed using methods found in U.S. Patent No. 9,388,453. One or more genes of the plasmid can be operatively linked to a promoter, such as an inducible promoter (which can be induced when packaging is initiated by inducing the bacteriophage). In some embodiments, the promoter can be a native promoter of a small terminase gene (*terS*) or a large terminase (*terL*) gene. The native promoter can be controlled by the bacteriophage, and thus effectively acts as a conditional promoter induced during packaging.

In some examples, it is preferable that the disruption/complementation is designed such that there is no homology between the mutated virus DNA and the complementing exogenous DNA. This is because lack of homology between the mutated virus DNA and the complementing exogenous DNA avoids the possibility of homologous recombination between the two DNA molecules that can result in re-introduction of a packaging sequence into the virus genome. To accomplish a lack of homology, one strategy is to delete the entire gene (or genes) that contains the packaging initiation site sequence from the virus genome and then complement this gene with an exogenous DNA molecule that preferably contains no more than exactly the DNA sequence that was deleted from virus. In this strategy, the complementing DNA molecule is designed to express the gene that was deleted from the virus. Another example of such a system is provided using the bacteriophage ϕ80α, a pac-type phage. The phage genome is lysogenized in a host bacterial cell, and the phage genome includes a small terminase gene where the pac-site of a pac-type prophage ϕ80α has been deleted. A plasmid including a complementary small terminase gene with a native pac-site is transformed into the cell. When the lytic cycle of the lysogenized prophage is induced, the bacteriophage packaging system packages plasmid DNA into progeny bacteriophage structural components, rather than packaging the native bacteriophage DNA. The packaging system thus produces non-replicative transduction particles carrying plasmid DNA.

The reporter gene encodes a detectable marker or a selectable marker. In an example, the reporter gene is selected from the group consisting of enzymes mediating luminescence reactions (LuxA, LuxB, LuxAB, Luc, Ruc, nLuc), enzymes mediating colorimetric reactions (LacZ, HRP), fluorescent proteins (GFP, eGFP, YFP, RFP, CFP, BFP, mCherry, near-infrared fluorescent proteins), affinity peptides (His-tag, 3X-FLAG), and selectable markers (ampC, tet(M), CAT, erm). In an embodiment, the reporter gene is *luxA.* In some embodiments, the resistance marker comprises an antibiotic resistance gene. In some embodiments, the resistance marker is a kanamycin resistance gene (kan). In some embodiments, the constitutive promoter comprises pBla (promoter for ampicillin resistance gene). In some embodiments, the bacteriophage genome disruption is accomplished by an allelic exchange event that replaces or disrupts a sequence on the bacteriophage genome that contains the packaging initiation site sequence.

In an example, a pair of terminase genes on a bacteriophage genome, *e.g., terS* and *terL,* can be disrupted in a manner that causes polar effects that also disrupt expression of one of the terminase genes and/or overall terminase function mediated by the terminase genes. The disrupted bacteriophage can be complemented with a plasmid comprising terminase genes, e.g., *terS* and *terL,* of the bacteriophage genome. When the mutated virus is undergoing a lytic cycle, the viral packaging proteins, produced either from the bacteriophage genome or (if disrupted) the complementing plasmid, package a replicon of the plasmid DNA into the packaging unit because it contains a packaging initiation site, and non-replicative transduction particles are produced carrying the replicated plasmid DNA.

### IV. Reporters

In some embodiments, the NRTPs and constructs of the invention comprise a reporter nucleic acid molecule including a reporter gene. The reporter gene can encode a reporter molecule, and the reporter molecule can be a detectable or selectable marker. In certain embodiments, the reporter gene encodes a reporter molecule that produces a detectable signal when expressed in a cell.

In certain embodiments, the reporter molecule can be a fluorescent reporter molecule, such as, but not limited to, a green fluorescent protein (GFP), enhanced GFP, yellow fluorescent protein (YFP), cyan fluorescent protein (CFP), blue fluorescent protein (BFP), red fluorescent protein (RFP) or mCherry, as well as near-infrared fluorescent proteins.

In other embodiments, the reporter molecule can be an enzyme mediating luminescence reactions (LuxA, LuxB, LuxAB, Luc, Ruc, nLuc, *etc*.). Reporter molecules can include a bacterial luciferase, a eukaryotic luciferase, an enzyme suitable for colorimetric detection (LacZ, HRP), a protein suitable for immunodetection, such as affinity peptides (His-tag, 3X-FLAG), a nucleic acid that function as an aptamer or that exhibits enzymatic activity (ribozyme), or a selectable marker, such as an antibiotic resistance gene (ampC, tet(M), CAT, erm). Other reporter molecules known in the art can be used for producing signals to detect target nucleic acids or cells.

In other aspects, the reporter molecule comprises a nucleic acid molecule. In some aspects, the reporter molecule is an aptamer with specific binding activity or that exhibits enzymatic activity (*e.g.,* aptazyme, DNAzyme, ribozyme).

Reporters and reporter assays are described further in Section V herein.

### V. NRTPs and Reporter Assays

### Inducer Reporter Assay

In some embodiments, the invention comprises methods for the use of NRTPs as reporter molecules for use with endogenous or native inducers that target gene promoters within viable cells. The NRTPs of the invention can be engineered using the methods described in Section III and further described in U.S. Patent No. 9,388,453.

In some embodiments, the method comprises employing a NRTP as a reporter, wherein the NRTP comprises a reporter gene that is operably linked to an inducible promoter that controls the expression of a target gene within a target cell. When the NRTP that includes the reporter gene is introduced into the target cell, expression of the reporter gene is possible via induction of the target gene promoter in the reporter nucleic acid molecule.

### Transcripts

As described above, a transcript is a length of nucleotide sequence (DNA or RNA) transcribed from a DNA or RNA template sequence or gene. The transcript can be a cDNA sequence transcribed from an RNA template or an mRNA sequence transcribed from a DNA template. The transcript can be transcribed from an engineered nucleic acid construct. The transcript can have regions of complementarity within itself, such that the transcript includes two regions that can form an intra-molecular duplex. One region can be referred to as a "cis-repressing sequence" that binds to and blocks translation of a reporter sequence. A second region of the transcript is called a "reporter sequence" that encodes a reporter molecule, such as a detectable or selectable marker.

The transcripts of the invention can be a transcript sequence that can be 5, 6, 7, 8, 9, 10, 11, 12, 13, 14, 15, 16, 17, 18, 19, 20, 21, 22, 23, 24, or 25 nucleotides in length. In other embodiments, the transcript can be at least 25, 30, 40, 50, 60, 70, 80, 90, 100, 500, 1000, 1500, 2000, 3000, 4000, 5000 or more nucleotides in length. The cis-repressing sequence and the reporter sequence can be the same length or of different lengths.

In some embodiments, the cis-repressing sequence is separated from the reporter sequence by 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, 35, 40, 45, 50, 55, 60, or more spacer nucleotides.

### Vectors

In another aspect, the transcripts (including antisense and sense sequences) of the invention are expressed from transcription units inserted into DNA or RNA vectors (*see, e.g.,* Couture, A, et al., TIG. (1996), 12:5-10; Skillern, A., et al., International PCT Publication No. WO 00/22113, Conrad, International PCT Publication No. WO 00/22114, and Conrad, U.S. Pat. No. 6,054,299). These sequences can be introduced as a linear construct, a circular plasmid, or a viral vector, including bacteriophage-based vectors, which can be incorporated and inherited as a transgene integrated into the host genome. The transcript can also be constructed to permit it to be inherited as an extrachromosomal plasmid (Gassmann, et al., Proc. Natl. Acad. Sci. USA (1995) 92:1292).

The transcript sequences can be transcribed by a promoter located on the expression plasmid. In one embodiment, the cis-repressing and reporter sequences are expressed as an inverted repeat joined by a linker polynucleotide sequence such that the transcript has a stem and loop structure.

Recombinant expression vectors can be used to express the transcripts of the invention. Recombinant expression vectors are generally DNA plasmids or viral vectors. Viral vectors expressing the transcripts can be constructed based on, but not limited to, adeno-associated virus (for a review, see Muzyczka, et al., Curr. Topics Micro. Immunol. (1992) 158:97-129)); adenovirus (see, for example, Berkner, et al., BioTechniques (1998) 6:616), Rosenfeld et al. (1991, Science 252:431-434), and Rosenfeld et al. (1992), Cell 68:143-155)); or alphavirus as well as others known in the art. Retroviruses have been used to introduce a variety of genes into many different cell types, including epithelial cells, *in vitro* and/or *in vivo* (see, *e.g.,* Eglitis, et al., Science (1985) 230:1395-1398; Danos and Mulligan, Proc. Natl. Acad. Sci. USA (1998) 85:6460-6464; Wilson et al., 1988, Proc. Natl. Acad. Sci. USA 85:3014-3018; Armentano et al., 1990, Proc. Natl. Acad. Sci. USA 87:61416145; Huber et al., 1991, Proc. Natl. Acad. Sci. USA 88:8039-8043; Ferry et al., 1991, Proc. Natl. Acad. Sci. USA 88:8377-8381; Chowdhury et al., 1991, Science 254:1802-1805; van Beusechem. et al., 1992, Proc. Natl. Acad. Sci. USA 89:7640-19 ; Kay et al., 1992, Human Gene Therapy 3:641-647; Dai et al., 1992, Proc. Natl. Acad. Sci. USA 89:10892-10895; Hwu et al., 1993, J. Immunol. 150:4104-4115; U.S. Patent No. 4,868,116; U.S. Patent No. 4,980,286; PCT Application WO 89/07136; PCT Application WO 89/02468; PCT Application WO 89/05345; and PCT Application WO 92/07573). Recombinant retroviral vectors capable of transducing and expressing genes inserted into the genome of a cell can be produced by transfecting the recombinant retroviral genome into suitable packaging cell lines such as PA317 and Psi-CRIP (Comette et al., 1991, Human Gene Therapy 2:5-10; Cone et al., 1984, Proc. Natl. Acad. Sci. USA 81:6349). Recombinant adenoviral vectors can be used to infect a wide variety of cells and tissues in susceptible hosts (*e.g.,* rat, hamster, dog, and chimpanzee) (Hsu et al., 1992, J. Infectious Disease, 166:769), and also have the advantage of not requiring mitotically active cells for infection.

Any viral vector capable of accepting the coding sequences for the transcript(s) to be expressed can be used, for example, vectors derived from adenovirus (AV); adeno-associated virus (AAV); retroviruses (*e.g.,* lentiviruses (LV), Rhabdoviruses, murine leukemia virus); herpes virus, and the like. The tropism of viral vectors can be modified by pseudotyping the vectors with envelope proteins or other surface antigens from other viruses, or by substituting different viral capsid proteins, as appropriate.

For example, lentiviral vectors featured in the invention can be pseudotyped with surface proteins from vesicular stomatitis virus (VSV), rabies, Ebola, Mokola, and the like. AAV vectors featured in the invention can be made to target different cells by engineering the vectors to express different capsid protein serotypes. Techniques for constructing AAV vectors which express different capsid protein serotypes are within the skill in the art; see, e.g., Rabinowitz J E et al. (2002), J Virol 76:791-801.

Selection of recombinant viral vectors suitable for use in the invention, methods for inserting nucleic acid sequences for expressing the transcripts into the vector, and methods of delivering the viral vector to the cells of interest are within the skill in the art. See, for example, Dornburg R (1995), Gene Therap. 2: 301-310; Eglitis M A (1988), Biotechniques 6: 608-614; Miller A D (1990), Hum Gene Therap. 1: 5-14; Anderson W F (1998), Nature 392: 25-30; and Rubinson D A et al., Nat. Genet. 33: 401-406.

Viral vectors can be derived from AV and AAV. A suitable AV vector for expressing the transcripts featured in the invention, a method for constructing the recombinant AV vector, and a method for delivering the vector into target cells, are described in Xia H et al. (2002), Nat. Biotech. 20: 1006-1010. Suitable AAV vectors for expressing the transcripts featured in the invention, methods for constructing the recombinant AV vector, and methods for delivering the vectors into target cells are described in Samulski R et al. (1987), J. Virol. 61: 3096-3101; Fisher K J et al. (1996), J. Virol, 70: 520-532; Samulski R et al. (1989), J. Virol. 63: 3822-3826; U.S. Pat. No. 5,252,479; U.S. Pat. No. 5,139,941; International Patent Application No. WO 94/13788; and International Patent Application No. WO 93/24641.

The promoter driving transcript expression in either a DNA plasmid or viral vector featured in the invention may be a eukaryotic RNA polymerase I (*e.g.,* ribosomal RNA promoter), RNA polymerase II (*e.g.,* CMV early promoter or actin promoter or U1 snRNA promoter) or generally RNA polymerase III promoter (*e.g.,* U6 snRNA or 7SK RNA promoter) or a prokaryotic promoter, for example the T7 promoter, provided the expression plasmid also encodes T7 RNA polymerase required for transcription from a T7 promoter. The promoter can also direct transgene expression to the pancreas (see, *e.g.,* the insulin regulatory sequence for pancreas (Bucchini et al., 1986, Proc. Natl. Acad. Sci. USA 83:2511-2515)).

In addition, expression of the transcript can be precisely regulated, for example, by using an inducible regulatory sequence and expression systems such as a regulatory sequence that is sensitive to certain physiological regulators, *e.g.,* circulating glucose levels, or hormones (Docherty et al., 1994, FASEB J. 8:20-24). Such inducible expression systems, suitable for the control of transgene expression in cells or in mammals include regulation by ecdysone, by estrogen, progesterone, tetracycline, chemical inducers of dimerization, and isopropyl-beta-D-1-thiogalactopyranoside (IPTG). A person skilled in the art would be able to choose the appropriate regulatory/promoter sequence based on the intended use of the dsRNA transgene. Generally, recombinant vectors capable of expressing transcript molecules are delivered as described below, and persist in target cells. Alternatively, viral vectors can be used that provide for transient expression of transcript molecules. Such vectors can be repeatedly administered as necessary. Once expressed, the transcript binds to target RNA and modulates its function or expression. Delivery of transcript expressing vectors can be systemic, such as by intravenous or intramuscular administration, by administration to target cells ex-planted from the patient followed by reintroduction into the patient, or by any other means that allows for introduction into a desired target cell.

Transcript expression DNA plasmids are typically transfected into target cells as a complex with cationic lipid carriers (*e.g.,* Oligofectamine) or non-cationic lipid-based carriers (*e.g.,* Transit-TKO^{™}). Multiple lipid transfections for dsRNA-mediated knockdowns targeting different regions of a single PROC gene or multiple PROC genes over a period of a week or more are also contemplated by the invention. Successful introduction of vectors into host cells can be monitored using various known methods. For example, transient transfection can be signaled with a reporter, such as a fluorescent marker, such as Green Fluorescent Protein (GFP). Stable transfection of cells *ex vivo* can be ensured using markers that provide the transfected cell with resistance to specific environmental factors (*e.g.,* antibiotics and drugs), such as hygromycin B resistance.

The delivery of the vector containing the recombinant DNA can by performed by abiologic or biologic systems. Including but not limited to electroporation (as described in the Examples), liposomes, virus-like particles, transduction particles derived from phage or viruses, and conjugation.

### Reporters for Transcript Assay

In some embodiments, the nucleic acid construct comprises a reporter sequence (*e.g.,* a reporter gene sequence). The reporter gene encodes a reporter molecule that produces a signal when expressed in a cell. In some embodiments, the reporter molecule can be a detectable or selectable marker. In certain embodiments, the reporter molecule can be a fluorescent reporter molecule, such as a green fluorescent protein (GFP), yellow fluorescent protein (YFP), cyan fluorescent protein (CFP), blue fluorescent protein (BFP), or red fluorescent protein (RFP). In other embodiments, the reporter molecule can be a chemiluminescent protein.

Reporter molecules can be a bacterial luciferase, an eukaryotic luciferase, a fluorescent protein, an enzyme suitable for colorimetric detection, a protein suitable for immunodetection, a peptide suitable for immunodetection or a nucleic acid that function as an aptamer or that exhibits enzymatic activity.

Selectable markers can also be used as a reporter. The selectable marker can be an antibiotic resistance gene, for example.

### Polymerase Chain Reaction (PCR)

U.S. Pat. Nos. 4,683,202, 4,683,195, 4,800,159, and 4,965,188 disclose conventional PCR techniques. PCR typically employs two oligonucleotide primers that bind to a selected nucleic acid template (e.g., DNA or RNA). Primers useful in some embodiments include oligonucleotides capable of acting as points of initiation of nucleic acid synthesis within the described target gene nucleic acid sequences. A primer can be purified from a restriction digest by conventional methods, or it can be produced synthetically. The primer is preferably single-stranded for maximum efficiency in amplification, but the primer can be double-stranded. Double-stranded primers are first denatured, i.e., treated to separate the strands. One method of denaturing double stranded nucleic acids is by heating.

If the template nucleic acid is double-stranded, it is necessary to separate the two strands before it can be used as a template in PCR. Strand separation can be accomplished by any suitable denaturing method including physical, chemical or enzymatic means. One method of separating the nucleic acid strands involves heating the nucleic acid until it is predominately denatured (e.g., greater than 50%, 60%, 70%, 80%, 90% or 95% denatured). The heating conditions necessary for denaturing template nucleic acid will depend, e.g., on the buffer salt concentration and the length and nucleotide composition of the nucleic acids being denatured, but typically range from about 90°C to about 105°C for a time depending on features of the reaction such as temperature and the nucleic acid length. Denaturation is typically performed for about 30 sec to 4 min (e.g., 1 min to 2 min 30 sec, or 1.5 min).

If the double-stranded template nucleic acid is denatured by heat, the reaction mixture is allowed to cool to a temperature that promotes annealing of each primer to its target sequence on the described target gene nucleic acid molecules. The temperature for annealing is usually from about 35°C to about 65°C (e.g., about 40°C to about 60°C; about 45°C to about 50°C). Annealing times can be from about 10 sec to about 1 min (e.g., about 20 sec to about 50 sec; about 30 sec to about 40 sec). The reaction mixture is then adjusted to a temperature at which the activity of the polymerase is promoted or optimized, i.e., a temperature sufficient for extension to occur from the annealed primer to generate products complementary to the template nucleic acid. The temperature should be sufficient to synthesize an extension product from each primer that is annealed to a nucleic acid template, but should not be so high as to denature an extension product from its complementary template (e.g., the temperature for extension generally ranges from about 40°C to about 80°C (e.g., about 50°C to about 70°C; about 60°C). Extension times can be from about 10 sec to about 5 min (e.g., about 30 sec to about 4 min; about 1 min to about 3 min; about 1 min 30 sec to about 2 min).

PCR assays can employ nucleic acid such as RNA or DNA (cDNA). The template nucleic acid need not be purified; it may be a minor fraction of a complex mixture, such as nucleic acid contained in human cells. Nucleic acid molecules may be extracted from a biological sample by routine techniques such as those described in Diagnostic Molecular Microbiology: Principles and Applications (Persing et al. (eds), 1993, American Society for Microbiology, Washington D.C.). Nucleic acids can be obtained from any number of sources, such as plasmids, or natural sources including bacteria, yeast, protozoa viruses, organelles, or higher organisms such as plants or animals.

The oligonucleotide primers are combined with PCR reagents under reaction conditions that induce primer extension. For example, chain extension reactions generally include 50 mM KCl, 10 mM Tris-HCl (pH 8.3), 15 mM MgCl2, 0.001% (w/v) gelatin, 0.5-1.0 µgprotodenatured template DNA, 50 pmoles of each oligonucleotide primer, 2.5 U of Taq polymerase, and 10% DMSO). The reactions usually contain 150 to 320 µM each of dATP, dCTP, dTTP, dGTP, or one or more analogs thereof.

The newly synthesized strands form a double-stranded molecule that can be used in the succeeding steps of the reaction. The steps of strand separation, annealing, and elongation can be repeated as often as needed to produce the desired quantity of amplification products corresponding to the target nucleic acid molecules. The limiting factors in the reaction are the amounts of primers, thermostable enzyme, and nucleoside triphosphates present in the reaction. The cycling steps (i.e., denaturation, annealing, and extension) are preferably repeated at least once. For use in detection, the number of cycling steps will depend, e.g., on the nature of the sample. If the sample is a complex mixture of nucleic acids, more cycling steps will be required to amplify the target sequence sufficient for detection. Generally, the cycling steps are repeated at least about 20 times, but may be repeated as many as 40, 60, or even 100 times. Fluorescence Resonance Energy Transfer (FRET) technology (see, for example, U.S. Pat. Nos. 4,996,143, 5,565,322, 5,849,489, and 6,162,603) is based on a concept that when a donor fluorescent moiety and a corresponding acceptor fluorescent moiety are positioned within a certain distance of each other, energy transfer takes place between the two fluorescent moieties that can be visualized or otherwise detected and/or quantitated. The donor typically transfers the energy to the acceptor when the donor is excited by light radiation with a suitable wavelength. The acceptor typically re-emits the transferred energy in the form of light radiation with a different wavelength. In certain systems, non-fluorescent energy can be transferred between donor and acceptor moieties, by way of biomolecules that include substantially non-fluorescent donor moieties (see, for example, US Pat. No. 7,741,467).

In one example, a oligonucleotide probe can contain a donor fluorescent moiety and a corresponding quencher, which may or not be fluorescent, and which dissipates the transferred energy in a form other than light. When the probe is intact, energy transfer typically occurs between the donor and acceptor moieties such that fluorescent emission from the donor fluorescent moiety is quenched the acceptor moiety. During an extension step of a polymerase chain reaction, a probe bound to an amplification product is cleaved by the 5' to 3' nuclease activity of, e.g., a Taq Polymerase such that the fluorescent emission of the donor fluorescent moiety is no longer quenched. Exemplary probes for this purpose are described in, e.g., U.S. Pat. Nos. 5,210,015, 5,994,056, and 6,171,785. Commonly used donor-acceptor pairs include the FAM-TAMRA pair. Commonly used quenchers are DABCYL and TAMRA. Commonly used dark quenchers include BlackHole Quenchers^{™} (BHQ), (Biosearch Technologies, Inc., Novato, Cal.), Iowa Black^{™}, (Integrated DNA Tech., Inc., Coralville, Iowa), BlackBerry^{™} Quencher 650 (BBQ-650), (Berry & Assoc., Dexter, Mich.).

Representative donor fluorescent moieties that can be used with various acceptor fluorescent moieties in FRET technology include fluorescein, Lucifer Yellow, B-phycoerythrin, 9-acridineisothiocyanate, Lucifer Yellow VS, 4-acetamido-4'-isothio-cyanatostilbene-2,2'-disulfonic acid, 7-diethylamino-3-(4'-isothiocyanatophenyl)-4-methylcoumarin, succinimdyl 1-pyrenebutyrate, and 4-acetamido-4'-isothiocyanatostilbene-2,2'-disulfonic acid derivatives. Representative acceptor fluorescent moieties, depending upon the donor fluorescent moiety used, include LC Red 640, LC Red 705, Cy5, Cy5.5, Lissamine rhodamine B sulfonyl chloride, tetramethyl rhodamine isothiocyanate, rhodamine x isothiocyanate, erythrosine isothiocyanate, fluorescein, diethylenetriamine pentaacetate, or other chelates of Lanthanide ions (e.g., Europium, or Terbium). Donor and acceptor fluorescent moieties can be obtained, for example, from Molecular Probes (Junction City, Oreg.) or Sigma Chemical Co. (St. Louis, Mo.).

As an alternative to FRET, an amplification product can be detected using a double-stranded DNA binding dye such as a fluorescent DNA binding dye (e.g., SYBR^{®} Green or SYBR^{®} Gold (Molecular Probes)). Upon interaction with the double-stranded nucleic acid, such fluorescent DNA binding dyes emit a fluorescence signal after excitation with light at a suitable wavelength. A double-stranded DNA binding dye such as a nucleic acid intercalating dye also can be used. When double-stranded DNA binding dyes are used, a melting curve analysis is usually performed for confirmation of the presence of the amplification product.

Melting curve analysis is an additional step that can be included in a cycling profile. Melting curve analysis is based on the fact that DNA melts at a characteristic temperature called the melting temperature (Tm), which is defined as the temperature at which half of the DNA duplexes have separated into single strands. The melting temperature of a DNA depends primarily upon its nucleotide composition. Thus, DNA molecules rich in G and C nucleotides have a higher Tm than those having an abundance of A and T nucleotides. By detecting the temperature at which signal is lost, the melting temperature of probes can be determined. Similarly, by detecting the temperature at which signal is generated, the annealing temperature of probes can be determined. The melting temperature(s) of the probes from the amplification products can confirm the presence or absence of the target strain/family of interest in the sample.

Within each thermocycler run, control samples can be cycled as well. Positive control samples can amplify target nucleic acid control template (other than described amplification products of target genes) using, for example, control primers and control probes. Positive control samples can also amplify, for example, a plasmid construct containing the target nucleic acid molecules. Such a plasmid control can be amplified internally (e.g., within the sample) or in a separate sample run side-by-side with the patients' samples using the same primers and probe as used for detection of the intended target. Such controls are indicators of the success or failure of the amplification, hybridization, and/or FRET reaction. Each thermocycler run can also include a negative control that, for example, lacks target template DNA. Negative control can measure contamination. This ensures that the system and reagents would not give rise to a false positive signal.

It is understood that the embodiments of the present disclosure are not limited by the configuration of one or more commercially available instruments.

### EXAMPLES

Below are examples of specific embodiments for carrying out the present invention. The examples are offered for illustrative purposes only, and are not intended to limit the scope of the present invention in any way. Efforts have been made to ensure accuracy with respect to numbers used (*e.g.,* amounts, temperatures, etc.), but some experimental error and deviation should, of course, be allowed for.

The practice of the present invention will employ, unless otherwise indicated, conventional methods of protein chemistry, biochemistry, recombinant DNA techniques and pharmacology, within the skill of the art. Such techniques are explained fully in the literature. See, *e.g.,* T.E. Creighton, Proteins: Structures and Molecular Properties (W.H. Freeman and Company, 1993); A.L. Lehninger, Biochemistry (Worth Publishers, Inc., current addition); Sambrook, et al., Molecular Cloning: A Laboratory Manual (2nd Edition, 1989); Methods In Enzymology (S. Colowick and N. Kaplan eds., Academic Press, Inc.); Remington's Pharmaceutical Sciences, 18th Edition (Easton, Pennsylvania: Mack Publishing Company, 1990); Carey and Sundberg Advanced Organic Chemistry 3rd Ed. (Plenum Press) Vols A and B(1992).

### Example 1: Overview of in silico qPCR assay design

### 1.1 Identify DNA sequences of phage terminase large subunit

Phage terminase proteins are responsible for DNA recognition and initiation of DNA packaging in phages. All identified viable phages harbor the terminase protein sequences in phage genome. Presence of consensus terminase sequences in the induced cell lysate is proposed to be an indicator of prophage in the lysate. The workflow of identification of DNA sequences of phage terminase large subunit is as following.
1. Download genome assemblies of *Klebsiella pneumoniae* (Kpn), *Escherichia coli* (Eco), *Enterobacter cloacae* from (Ecl) from NCBI database.
2. Identify amino acid sequences of terminase for each of the three bacteria from genome assemblies by annotation.
3. Separate the identified amino acid sequences of terminase into three groups by annotation, large subunit, small subunit and unknown subunit respectively.
4. Classify the sequences of unknown subunit into large subunit and small subunit by blast searching the sequences against sequences of large subunit and small subunit identified from previous step. The classification of a sequence of unknown subunit was based on its best similarity to a sequence of large subunit or small subunit.
5. Identify cDNA sequences of terminase large subunit from genome assemble by annotation search.
6. Cluster the cDNA sequences into multiple clades for each of the bacterial species respectively.
7. Design qPCR assay for each of the clades.

**Table I**

| **Consensus sequences as primers for Kpn prophage clades identification** | | |
|---|---|---|
| **Clades** | **Consensus Sequence 1** | **Consensus Sequence 2** |
| Clade01 | gggctatcacatatcacgtttgtac (SEQ ID NO:1) | acaaaacgcatatggtcaaagttcttaaa (SEQ ID NO: 13) |
| Clade02 | tgcaaggtggtggtgacc SEQ ID NO: 2 | ggatcgttgctgtacatcgc (SEQ ID NO: 14) |
| Clade03 | atgaagcctgagcatctcaa (SEQ ID NO: 3) | tgtCgGtgatccAgtaAagaTtgttc (SEQ ID NO: 15) |
| Clade04 | actccgtcccgtggga (SEQ ID NO: 4) | tgtagacaatccagccatcaatca (SEQ ID NO: 16) |
| Clade05 | gacaatttgtcggtcgatggaaa (SEQ ID NO: 5) | cgcgattatttttcgccacgc (SEQ ID NO: 17) |
| Clade06 | gcctgaatatgtcatccaccaac (SEQ ID NO: 6) | gacaccgtcgatttgaatctcttc (SEQ ID NO: 18) |
| Clade07 | cgccgggatccgga (SEQ ID NO: 7) | eg aaattaagg a etttgtggg eateaa (SEQ ID NO: 19) |
| Clade08 | ggcgaaattccggcctgtaa (SEQ ID NO: 8) | ggagaacgcaataaaccgctc (SEQ ID NO: 20) |
| Clade09 | tgctggtggagttctggac (SEQ ID NO: 9) | gcaacaaagccatatttcaccgc (SEQ ID NO: 21) |
| Clade10 | ggcaacctatccgaacgtca (SEQ ID NO: 10) | atgtcgctgacaggcaagta (SEQ ID NO: 22) |
| Clade11 | actacgatgatttgcgtgggtc (SEQ ID NO: 11) | accgacaagtcgacagcatc (SEQ ID NO: 23) |
| Clade12 | tggtatctggcgatatcgttgc (SEQ ID NO: 12) | cgcggccttcatcaaaataaaca (SEQ ID NO: 24) |

**Table II**

| **Consensus sequences as primers for Ecl prophage clades identification** | | |
|---|---|---|
| **Clades** | **Consensus Sequence 1** | **Consensus Sequence 2** |
| Clade02 | cgggaagatatgcgccaagta (SEQ ID NO: 25) | tggcagtaatcgaagaagtaaccc (SEQ ID NO: 35) |
| Clade03 | ggaagagcagctcgtcga (SEQ ID NO: 26) | gccagctctgtgccctc (SEQ ID NO: 36) |
| Clade04 | gggatcagcccgacactta (SEQ ID NO: 27) | cgccacaacccacccac (SEQ ID NO: 37) |
| Clade05 | tgcaggaggtgaaacaggc (SEQ ID NO: 28) | cgaggttatgccgcagacc (SEQ ID NO: 38) |
| Clade06 | tgcgcagttagccaggttc (SEQ ID NO: 29) | ccgtcgttgaacaggcga (SEQ ID NO: 39) |
| Clade07 | cctgccctgactgggc (SEQ ID NO: 30) | atcaacgattttcagttgcctgaa (SEQ ID NO: 40) |
| Clade08 | gatggcatggtcgcaatgac (SEQ ID NO: 31) | cagcggtcggaaaatgaagtc (SEQ ID NO: 41) |
| Clade09 | acagtcacgacaatgacgaca (SEQ ID NO: 32) | tgagttggctcgggtcgata (SEQ ID NO: 42) |
| Clade10 | ggcgattttgttcgccatcac (SEQ ID NO: 33) | acctcgctttcgtcgtcc (SEQ ID NO: 43) |
| Clade11 | ttgcagaaggcatccgcta (SEQ ID NO: 34) | cactgaagaaaataccgcgctc (SEQ ID NO: 44) |

**Table III**

| **Consensus sequences as primers for Eco prophage clades identification** | | |
|---|---|---|
| **Clades** | **Consensus Sequence 1** | **Consensus Sequence 2** |
| CladeM01 | cgggagtaccgtaaagccc (SEQ ID NO: 45) | ccctgctgctcccagaaatc (SEQ ID NO: 63) |
| CladeM02 | ccgatttatccggaccaggc (SEQ ID NO: 46) | ccgccaccaggtcaaaca (SEQ ID NO: 64) |
| CladeM03 | accaggccgcacatgc (SEQ ID NO: 47) | ccaccaggtcaaacaccca (SEQ ID NO: 65) |
| CladeM04 | ggagccgttcccgcaa (SEQ ID NO: 48) | tcgcgtcactctgcatcac (SEQ ID NO: 66) |
| CladeM05 | ctgtattgttttgcctgtgataacga (SEQ ID NO: 49) | acctcaatcccgaacgcttc (SEQ ID NO: 67) |
| CladeM06 | cgcgccgggcttga (SEQ ID NO: 50) | gcgcctcgcgggaaaaataa (SEQ ID NO: 68) |
| CladeM07 | ccggtccgtgggagtca (SEQ ID NO: 51) | tgccgtacacaatccagcc (SEQ ID NO: 69) |
| CladeM08 | tcaggaaaccgcaaaggca (SEQ ID NO: 52) | gccaccagcggatccca (SEQ ID NO: 70) |
| CladeM09 | cgcgtcagcccggaa (SEQ ID NO: 53) | ggccagccgattttcaggta (SEQ ID NO: 71) |
| CladeM10 | gccagcatggagcaacga (SEQ ID NO: 54) | cctgaaccacaaaacggcga (SEQ ID NO: 72) |
| CladeM11 | tgcttgatggtcgcttcaaagta (SEQ ID NO: 55) | cgcatcatgtatgcatagcgaa (SEQ ID NO: 73) |
| CladeM12 | gctgttccgggtgatccc (SEQ ID NO: 56) | ccgtaccgatttcggtaaggtaaac (SEQ ID NO: 74) |
| CladeM13 | cgcagattcagcgcaaattcaa (SEQ ID NO: 57) | cgcatcagccaggttaggaga (SEQ ID NO: 75) |
| CIadeM14 | gcaggaaaaaaatactgtgggaca (SEQ ID NO: 58) | ctgccagcgggaaatactga (SEQ ID NO: 76) |
| CladeM15 | ctggacggcgtacagtcc (SEQ ID NO: 59) | cccagcgtggtgttcacaa (SEQ ID NO: 77) |
| CladeM16 | agacagaggcgcagcaac (SEQ ID NO: 60) | gcacggacacacgtaatgcc (SEQ ID NO: 78) |
| CladeM17 | actaccgtgaaaaatccgtgga (SEQ ID NO: 61) | ccatacagagccctcaatacgtttatc (SEQ ID NO: 79) |
| CladeM18 | g ctg eg catcagtatttccc (SEQ ID NO: 62) | cattcatcctctccggataaggc (SEQ ID NO: 80) |

### 1.2 qPCR assay design

The qPCR assays were designed by Roche in-house software. Design workflow includes major steps as following.
1. Create multiple alignments of sequences in each clade.
2. Call consensus sequence from the multiple alignments.
3. Design primers with melting temperature approximate 63°C based on the consensus sequences.
4. Design assays by combination of two primers (Forward+Reverse) with amplicon size between 70 to 150 bases.
5. Score assays to predict wet-lab performance
6. Select top candidate assays based on assay scores.

### 1.3. Selection of candidate assays

In general, the in-house software produces many assay designs with predictions of experimental performance. Top candidate assays were selected for experimental validation based on predicted merits such as inclusivity, probability of forming primer dimers, probability of forming G-quadraplex, GC content of amplicon, secondary structure of amplicon, lengths of amplicon and multiplexing compatibility of a panel of assays et al.

### Induction conditions for inducible prophage

Induction Protocol:
Day 1: Start an overnight culture plate from frozen cell stock. Inoculate 500uL TSB with 10uL thawed cell stock in a 2mL deep-well plate. Incubate at 37oC overnight at 600RPM shaking.
Day 2: Subculture the overnight culture at 1:50 dilution with Tryptic Soy Broth into 2 fresh 2mL deep-well plates and incubate at 37oC at 600RPM for 90min.

Induce 1 culture plate with 0.5-2 ug/ml mitomycin C for 4 hours at 37oC without shaking.

Spin down culture plates at ~4500g for 10 min.

Filter the supernatants through a 0.2um plate filter.

Store lysates at 4°C wrapped in foil or away from light.

PCR conditions used in Screening are shown in TABLE IV and TABLE V.

**TABLE IV PCR Amplification Reagents**

| ***Master Mix Component*** | ***Final Conc (45.0uL)*** | | | |
|---|---|---|---|---|
| DMSO | | 5.4 | | % |
| NaN3 | | 0.027 | | % |
| Potassium acetate | | 120.0 | | mM |
| Glycerol | | 3.0 | | % |
| Tween 20 | | 0.015 | | % |
| NaN3 | | 0.027 | | % |
| Tricine | | 60.0 | | mM |
| NTQ21-46AAptamer | | 0.222 | | µM |
| UNG Enzyme | | 10.0 | | U |
| Z05D-DNA Polymerase | | 45.0 | | U |
| dATP | | 400.0 | | µM |
| dCTP | | 400.0 | | µM |
| dGTP | | 400.0 | | µM |
| dUTP | | 800.0 | | µM |
| Forward primer oligonucleotides | | 0.50 | | µM |
| Reverse primer oligonucleotides | | 0.50 | | µM |
| Manganese Acetate | | 3.30 | | mM |
| Trizma Base | | 12 | | µM |
| Methylparaben | | 0.08 | | % |

**TABLE V PCR Thermoprofile**

| Program Name | Target (°C) | Acquisition Mode | Hold (hh:mm:ss) | Ramp Rate (°C / s) | Cycles | Analysis Mode |
|---|---|---|---|---|---|---|
| 1st Measurement | 95 | None | 00:00:05 | 4.4 | 5 | Quantification |
| | 55 | Single | 00:00:30 | 2.2 | | |
| 2nd Measurment | 91 | None | 00:00:05 | 4.4 | 45 | Quantification |
| | 58 | Single | 00:00:25 | 22 | | |
| Cooling | 40 | None | 00:02:00 | 2.2 | 1 | None |

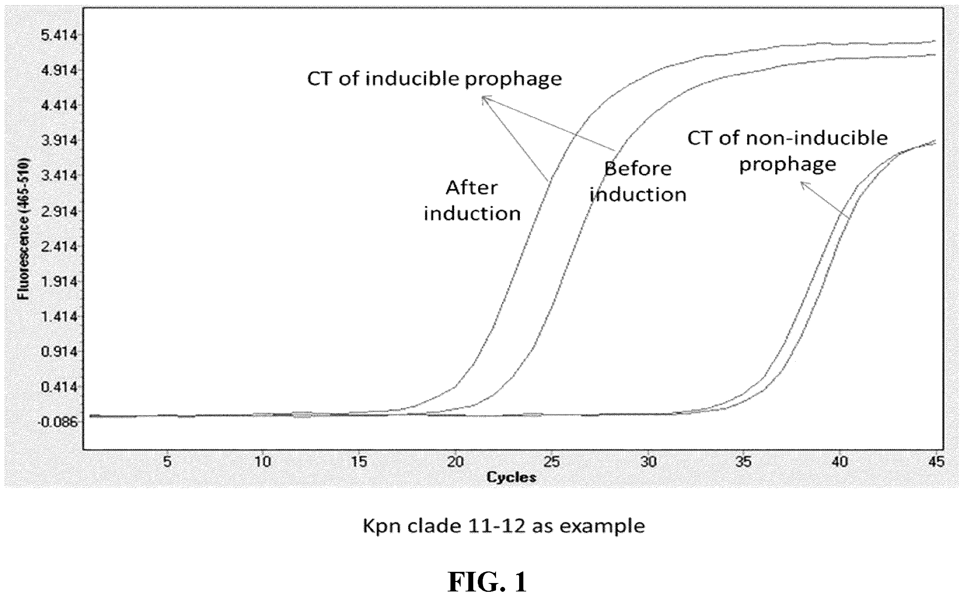

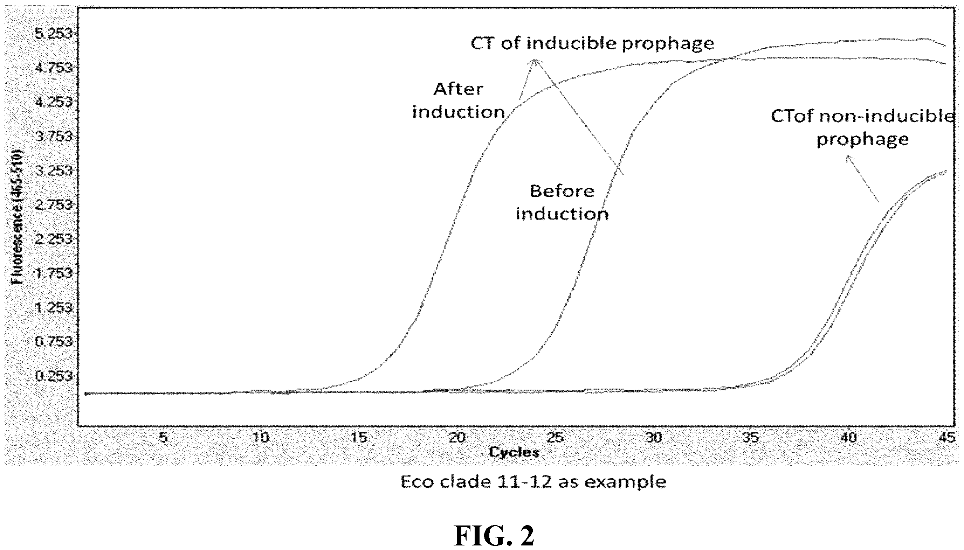

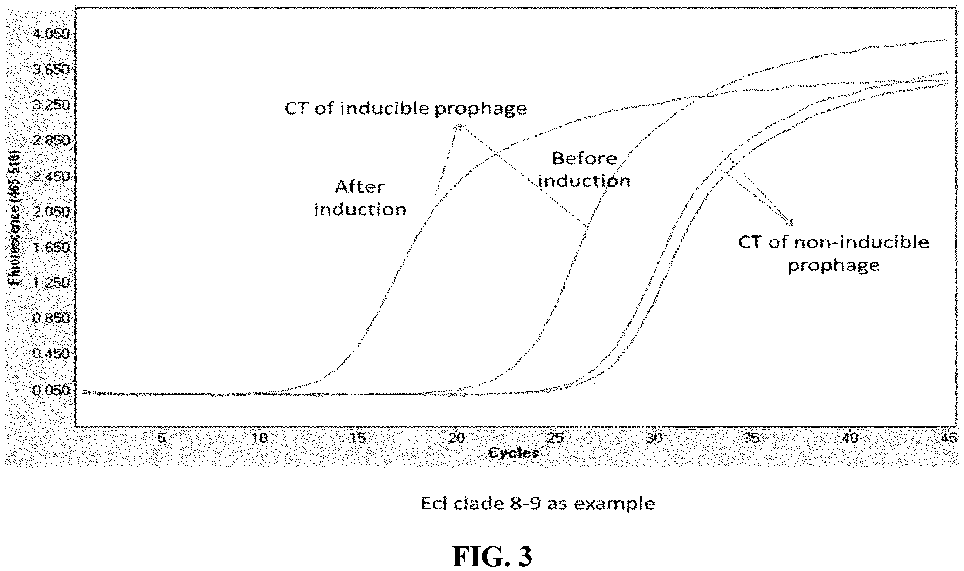

Dye is 0.2x SYBR (Roche, Penzburg) instead of a hydrolysis probe
DeltaCT cutoff
For positive hits: ΔCp > 1; Melting temperature: 81°C < Tₘ,Amplicon < 89°C
Examples of growth curves generated from actual qPCR experiments are shown on FIG. 1 for Kpn, on FIG. 2 for Eco and on FIG. 3 for Ecl.

### Example 2: Confirmation of Prophage candidates

qPCR identified prophages were sequenced via the MiSeq System (Illumina, San Diego), and the viability of the prophages were further confirmed with functional plaquing assay.

**TABLE VI MiSeq and plaquing assay evaluating Kpn prophages identified via qPCR method**

| **Kpn Strain ID** | **MiSeq identification** | **PFU Host Range** |
|---|---|---|
| Kpn 51 | Monolysogen | 34% (30/89) |
| Kpn 186 | Monolysogen | 39% (35/89) |
| Kpn 109 | Monolysogen | 36% (32/89) |
| Kpn 512 | Monolysogen | 18% (17/95) |

### Example 3: Reporter plasmid to generate NRTP using the phage genome information

### Kpn package cell line plasmid construction:

The plasmid was constructed to comprise of the following elements: a replicon for packaging plasmid replication, a hygromycin B resistance gene (hph), a reporter gene, and terSL including the cos-site to complement the phage function and to facilitate the plasmid packaging.

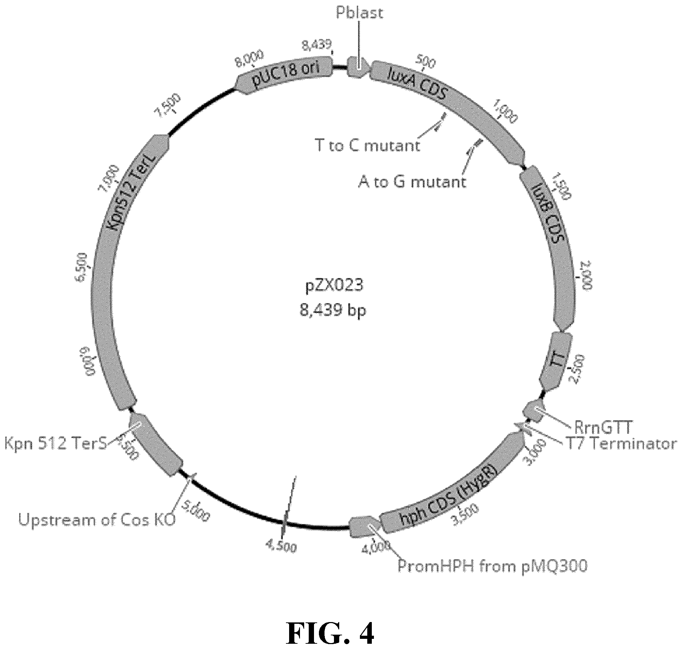

All the elements were cloned onto the backbone of pUC19. Mutant luxAB (C170R N264D) was cloned into pZX023 (FIG. 4) under the control of a pBla promoter.

The hph cassette encoding hygromycin B resistance was chosen as the final selective marker in the packaging plasmid. pBla promoter: Pbla was selected as the final promoter to drive the luxAB reporter genes expression. The Pbla promoter gene was subcloned from the ampicillin resistance gene and fused with luxAB-encoding genes. luxAB/eluxAB: The luxA and luxB genes are from Vibrio harveyi. These genes were originally obtained from work done in 1990 by Farinha et.al. Two single base mutations were engineered in luxA (C170R, N264) for plasmid pXZ023. The mutant LuxAB (i.e., eluxAB) protein has improved luciferase activity. terSL: The putative terSL was annotated via PhageTerm. The terSL genes were amplified via PCR from Kpn512 bacteriophage. 345bp DNA up stream of the terminase gene, including a putative cos site, and 172bp of downstream terL gene were also cloned into the packaging plasmid. T7-terminator and RrnGTT: T7 and RrnG terminators are cloned downstream of hph gene to stabilize the plasmid.

Plasmid pZX023 Sequence:

gaagatcctttgatcttttctacggggtctgacgctcagtggaacgaaaactcacgttaagggattttggtcatgagattatcctcgtcagg
tggcacttttcggggaaatgtgcgcggaacccctatttgtttatttttctaaatacattcaaatatgtatccgctcatgagacaataaccctgat
aaatgcttcaataatattgaaaaaggaagagtcgatcgatgaagtttggaaatatttgtttttcgtatcaaccaccaggtgaaactcataagc
aagtaatggatcgctttgttcggcttggtatcgcctcagaagaggtagggtttgatacatattggaccttagaacatcattttacagagtttg
gtcttacgggaaatttatttgttgctgcggctaacctgttaggaagaactaaaacattaaatgttggcactatgggggttgttattccgacag
cacacccagttcgacagttagaagacgttttattattagatcaaatgtcgaaaggtcgttttaattttggaaccgttcgagggctataccata
aagattttcgagtatttggtgttgatatggaagagtctcgagcaattactcaaaatttctaccagatgataatggaaagcttacagacagga
accattagctctgatagtgattacattcaatttcctaaggttgatgtatatcccaaagtgtactcaaaaaatgtaccaacccgtatgactgctg
agtccgcaagtacgacagaatggctagcaatacaagggctaccaatggttcttagttggattattggtactaatgaaaaaaaagcacaga
tggaactctataatgaaattgcgacagaatatggtcatgatatatctaaaatagatcattgtatgacttatatttgttctgttgatgatgatgca
caaaaggcgcaagatgtttgtcgggagtttctgaaaaattggtatgactcatatgtaaatgcgaccaatatctttaatgatagcgatcaaac
tcgtggttatgattatcataaaggtcaatggcgtgattttgttttacaaggacatacaaacaccaatcgacgtgttgattatagcaatggtatt
aacccCgtaggcactcctgagcagtgtattgaaatcattcaacgtgatattgatgcaacgggtattacaaacattacatgcggatttgaag
ctaatggaactgaagatgaaataattgcttccatgcgacgctttatgacacaagtcgctcctttcttaaaagaacctaaataaattacttattt
gatactagagataataaggaacaagttatgaaatttggattattttttctaaactttcagaaagatggaataacatctgaAgaaacgttggat
aatatggtaaagactgtcacgttaattgattcaactaaatatcattttaatactgcctttgttaatgaacatcacttttcaaaaaatggtattgttg
gagcacctattaccgcagctggttttttattagggttaacaaataaattacatattggttcattaaatcaagtaattacccatcaccctgta
cgtgtagcagaagaagccagtttattagatcaaatgtcagagggacgcttcattcttggttttagtgactgcgaaagtgatttcgaaatgga
attttttagacgtcatatctcatcaaggcaacaacaatttgaagcatgctatgaaataattaatgacgcattaactacaggttattgCcatcc
ccaaaacgacttttatgattttccaaaggtttcaattaatccacactgttacagtgagaatggacctaagcaatatgtatccgctacatcaaa
agaagtcgtcatgtgggcagcgaaaaaggcactgcctttaacGtttaagtgggaggataatttagaaaccaaagaacgctatgcaattc
tatataataaaacagcacaacaatatggtattgatatttcggatgttgatcatcaattaactgtaattgcgaacttaaatgctgatagaagtac
ggctcaagaagaagtgagagaatacttaaaagactatatcactgaaacttaccctcaaatggacagagatgaaaaaattaactgcattatt
gaagagaatgcagttgggtctcatgatgactattatgaatcgacaaaattagcagtggaaaaaacagggtctaaaaatattttattatccttt
gaatcaatgtccgatattaaagatgtaaaagatattattgatatgttgaaccaaaaaatcgaaatgaatttaccataaggatcctaaaattaa
aggcaatttctatattagattgcctttttggcgcgcctattctaatgcataataaatactgataacatcttatattttgtattatattttgtattatcgt
tgacatgtataattttgatatcaaaaactgattttccctctattattttcgagatttattttcttaattctctttaacaaactagaaatattgtatataca
aaaaattataaataatagatgaatagtttaattataggtgttcatcaatcgaaaaagcaacgtatcttatttaaagtgcgttgcttttttctcattt
ataaggttaaataattctcatatatcaagcaaagtgacagagctcggtactctcggcttgaacgaattggcggccgccctgcaggatagc
tgacgaccttaaggataaatttctggtaaggaggacacgtatggaagtgggcaagttggggaagccgtatccgttgctgaatctggcat
atgtgggagtataagacgcgcagcgtcgcatcaggcatttttttctgcgccaatgccggtagatagcaatccggcccgaggggcacaa
aaaacccctcaagacccgtttagaggccccaaggggttatgctacgatactcaggcgccgggggcggtgtccggcggcccccagag

gaactgcgccagttcctccggatcggtgaagccggagagatccagcggggtctcctcgaacacctcgaagtcgtgcaggaaggtga
aggcgagcagttcgcgggcgaagtcctcggtccgcttccactgcgccccgtcgagcagcgcggccaggatctcgcggtcgccccg
gaaggcgttgagatgcagttgcaccaggctgtagcgggGgtctcccgcatagacgtcggtgaagtcgacgatcccggtgacctcgg
tcgcggccaggtccacgaagatgttggtcccgtgcaggtcgccgtggacgaaccggggttcgcggccggccagcagcgtgtccac
gtccggcagccagtcctccaggcggtccagcagccggggcgagaggtagccccacccgcggtggtcctcgacggtcgccgcgcg
gcgttcccgcagcagttccgggaagacctcggaatggggggtgagcacggtgttcccggtcagcggcaccctgtgcagccggccg
agcacccggccgagttcgcgggccagggcgagcagcgcgttccggtcggtcgtgccgtccatcgcggaccgccaggtggtgccg
gtcaCccggctcatcaccaggtagggccacggccaggctccggtgccgggccgcagctcgccgcggccgaggaggcggggcac
cggcaccggggcgtccgccaggaccgcgtacgcctccgactccgacgcgaggctctccggaccgcaccagtgctcgccgaacag
cttgatcaccgggCcgggctcgccgaccagtacggggttggtgctctcgccgggcacccgcagcaccggcggcaccggcagccc
gagctcctccagggctcggcgggccagcggctcccagaattcctggtcgttccgcaggctcgcgtaggaatcatccgaatcaatacg
gtcgagaagtaacagggattcttgtgtcatatgaatcttactcctttgttaaattatttttgtttaagcattttgaatttgtatgcatcttagaacaa
tctcaaagttccaagctcttgcaagtctagatattgagtcaaaaaaaattttcaactcatactcttcctttttcaatattattgaagcatttatcag
ggttattgtctcatgagcgcacatttccccgaaaagtgccacctgacgtctaagaaaccattattatcatgacattaacctataaaaatagg
cgtatcacgaggccctttcgtctcgcgcgtttcggtgatgacggtgaaaacctctgacacatgcagctcccggagacggtcacagcttg
tctgtaagcggatgccgggagcagacaagcccgtcagggcgcgtcagcgggtgttggcgggtgtcggggctggcttaactatgcgg
catcagagcagattgtactgagagtgcaccatatgcggtgtgaaataccgcacagatgcgtaaggagaaaataccgcatcaggcgcc
attcgccattcaggctgcgcaactgttgggaagggcgatcggtgcgggcctcttcgctattacgccagctggcgaaagggggatgtgc
tgcaaggcgattaagttgggtaacgccagggttttcccagtcacgacgttgtaaaacgacggccagtgAATTCGAGCTCGG
TACCCGGGGATCCTCTAGAGTCGACCTGCAGGCATGCAAGCTTGGCgtaatcatggtcata
gctgtttcctgtgtgaaattgttatccgctcacaattccacacaacatacgagccggaagcataaagtgtaaagcctggggtgcctaatga
gtgagctcagcaggcattacaacaggcgctcacagagcgcctgtgataatggctgaatgcttcaccagcgcggcgttttatgggaggt
catgatgagttacaccagctgtacttattgcggttcacgtctccatacgcgggctaactgcccgaaaacatggggcggttcatcgcgtcg
cgccaatctgcgctgcagctattgtggccagtccgggcataactcgaatgcctgcccacataatgcgagcagcagtcggcggcgcag
cctgaatgacgacttccacctcgactgaggcagccgccgtcatggggtaggggggatcaaatccctaacccctttcgcgcttcgggac
tgccgcttcaggtagatttttgcgcgtgagaaataaaaactttttttttggctgttttggggtgcttttgaatgagtacagggatgcgatcacc
tggtggcggacgcaaatcgaataacactggaaatcaggttagttctttaaccagagcggtttctccgccggatgaattactgggcgatat
ggctatcgatgcctggaaacggacgtgcaaaattcttattaaccgtggcacgttcgaaatggaagattgttatttgctgatggaatactgc
aacaccgtgcagctgctgtacgacgccaaccaggaaattaaaagcgatggccttggtgatgataccgctgccggcggtcagaaacttg
gtgcggcagtgaaggcgcgtagccgttatatcagcgaattaattcgactctccgttgtgttaaagctggaccccaatagccgcatcctga
agaaacagcccggagataatgacaaatccagcggtgagttcgacgagttttaattttggtgcggccctaatgacttaaggatggagcat
ggccgcatatccaaacgtcaatgtggcgaacaaatatgcgcgggatatcatagacgggaaaatagtcgcctgcagagctattcggctg
gcatgtcagcgccattttgacgatttaaaaaaatcactcgataacaataacccttaccggttcgacagagatttagctgagcgggcctgcc
ggtttgttcagaagttaccgcactccagtggcgatttggcgggacagaaattaaaactggaaccttggcaaagttttattttttgttcgatttt

tggctgggtcacgaaaaaggataaaaaacgccgatttcgcgaagcgtatatccgggtagctaggaaaaacgggaaatcgttttttgctg
ccgggattggcacctacatgttttgcgctgatggcgaaaacagtgcagaagtgtattgcggtgcgacaactatggcgcaggcgaaaaa
ggtcttcaccccagccaggcagatggccagccgcctgccggcacttcgctccagatttgatatttcggtatggaccgacagcctgacac
gcccggatggttccgttttcgcacctatggcggggaaacccggcgatggtgacagcccacattgcgcgatcattgacgagtatcacga
acacgatacggatcatatgtacgaggccatgacaatggggatgggcgcccgttcgcagccgttaacgctcattatcacgacagccggc
tcgtcactggagtcaccttgctatgacaaggacaaggaagtcaaagaggttatcgaaggcataacccgtaatgatcgcctgtttggcatg
atttacgaactggatgctggcgatgactggaccgacccgaaaaacttaatcaaagctaacccaaatctggacgtttcggttaagtacagc
gacctggttgagcttctggaagtagcgaaacaggttcctcgcaaggttaacgccttcaaaaccaaacgcctcaatatttgggtatccggt
aaatccgcgttctacaacatggagcagtggaaggctgctgaagaccccgaccttgagctggctgattttgcgaatgacagctgcaacat
cggtctcgatctcgccaaaaagctggatatgaacgccgggatacggctatttacgcgggaaattgaaggtaaacggcattattactgcat
caaacctaaattttgggtcccggaagacactatccatacaaccgatccaaaactgctgaaaactgctgacaggtatcagaagttttatgaa
atgggcgtgctggaagcgacggatggcgcagaggcagactatcgcgagattctggccagtattatcgatatgcaggacgaaaaccg
cattgacgagattgatatcgaccctgccggcgcaacagcacttcgccaccagttggaggacaacggatttaccgtagtcgatatccggc
aggattacaccaatatgtcaccggcgatgaaagagcttgaagcggctctggccggtggtcgattccaccatgatggcaatcccattctg
acctggtgtatcagcaacgttatcgggaaatttatacccggtagcgatgatctcgttcgcccgacaaagggagacaatcaaagcaaaat
cgatggagctacagcgttatttaacgccatgactcgcgcaatgctgcacgaaagcagcggcggcacatcggtatatgatgaggaaga
catagcgtgttaatcacaattctgagtttcattattggcctggccggggctgtactcatatccgccggagcctggttgattttgcctgctgcc
ggtcttattacgggcgggtcaatatgtcttatctggtcatatctgactgcgcgggcggtttcagccggtgccaaatttaacgggggtgaat
aactcacattaattgcgttgcgctcactgcccgctttccagtcgggaaacctgtcgtgccagctgcattaatgaatcggccaacgcgcgg
ggagaggcggtttgcgtattgggcgctcttccgcttcctcgctcactgactcgctgcgctcggtcgttcggctgcggcgagcggtatca
gctcactcaaaggcggtaatacggttatccacagaatcaggggataacgcaggaaagaacatgtgagcaaaaggccagcaaaaggc
caggaaccgtaaaaaggccgcgttgctggcgtttttccataggctccgcccccctgacgagcatcacaaaaatcgacgctcaagtcag
aggtggcgaaacccgacaggactataaagataccaggcgtttccccctggaagctccctcgtgcgctctcctgttccgaccctgccgct
taccggatacctgtccgcctttctcccttcgggaagcgtggcgctttctcatagctcacgctgtaggtatctcagttcggtgtaggtcgttc
gctccaagctgggctgtgtgcacgaaccccccgttcagcccgaccgctgcgccttatccggtaactatcgtcttgagtccaacccggta
agacacgacttatcgccactggcagcagccactggtaacaggattagcagagcgaggtatgtaggcggtgctacagagttcttgaagt
ggtggcctaactacggctacactagaagAacagtatttggtatctgcgctctgctgaagccagttaccttcggaaaaagagttggtagct
cttgatccggcaaacaaaccaccgctggtagcggtggtttttttgtttgcaagcagcagattacgcgcagaaaaaaaggatctcaa
(SEQ ID NO: 81)

### Example 4: Inducible Prophage from Pseudomonas aeurosinosa

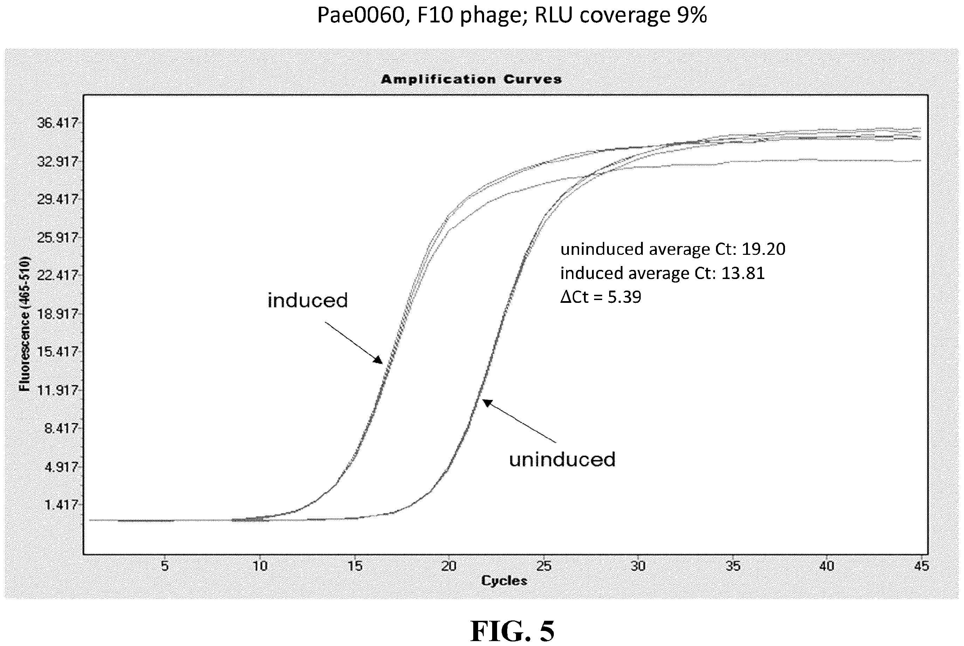

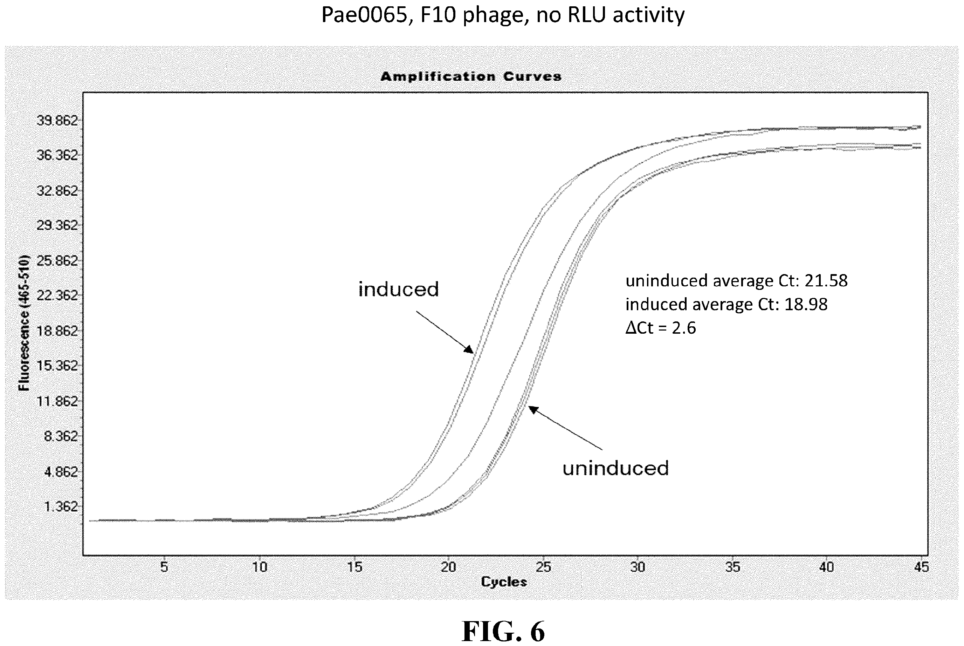

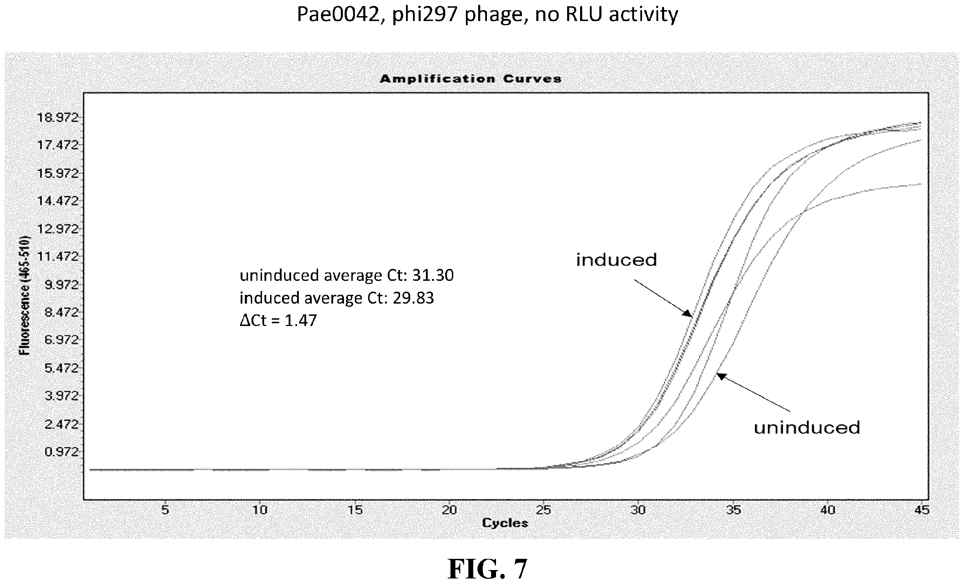

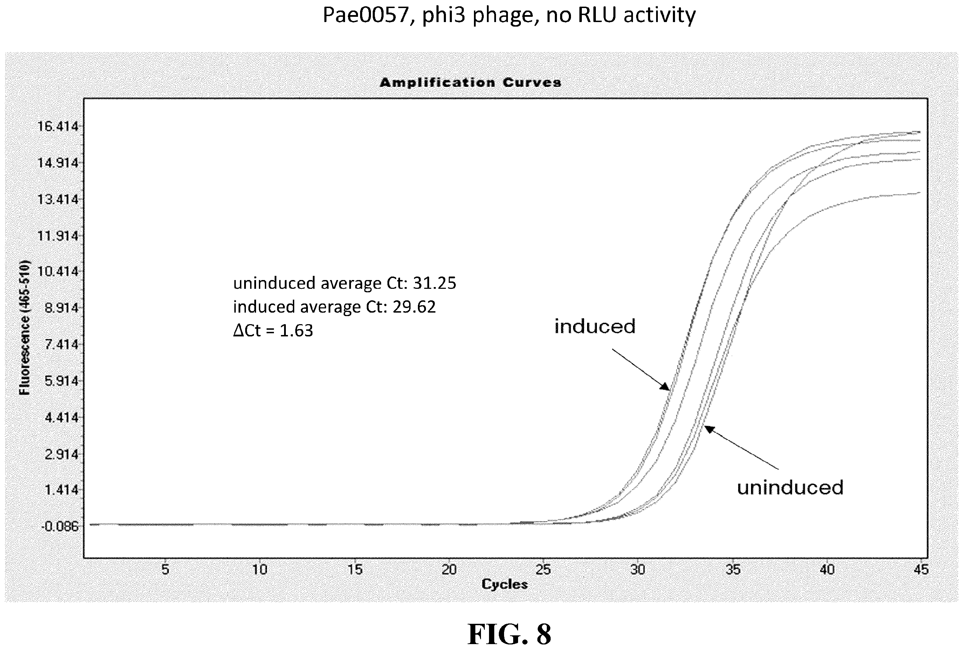

Oligonucleotide primers were designed that would recognize the phage terminase large subunit of *Pseudomonas aeruginosa* (Pae). The nucleotide sequences of these primers and their types are shown below (TABLE VII) and the growth curves of the qPCR experiments (using the conditions as described in Example 1) are shown on FIGs. 5-8. Based on the observed ΔCt values following induction or non-induction of each Pae strain, it was determined that only the prophages from Pae strain 0060 (with a ΔCt between induced and non-induced of over 5) were able to generate functional NRTPs as expressed in light generation from the luciferase gene as relative light units (RLU).

**TABLE VII Primers for prophage identification in Pae strains**

| **Oligonucleotide Name** | **Primer Type** | **Phage Type** | **Sequence** |
|---|---|---|---|
| Pae060_F10 terL_F | forward | F10 | acgactgccattggatgtgt (SEQ ID NO: 82) |
| Pae060_F10 terL_R | reverse | F10 | tgctgtacacccttgacgac (SEQ ID NO: 83) |
| Pae065_F10 terL_F | forward | F10 | tccaagctgacctatgcacg (SEQ ID NO: 84) |
| Pae065_F10 terL_R | reverse | F10 | gatccaccgagtagcccatg (SEQ ID NO: 85) |
| Pae042_phi297 terL_F | forward | Phi297 | gcgactgatcatcaacgtgc (SEQ ID NO: 86) |
| Pae042_phi297 terL_R | reverse | Phi297 | cctgatacgccgaggacttc (SEQ ID NO: 87) |
| Pae057_phi3 terL_F | forward | Phi3 | atatcctgaagtcgcgccag (SEQ ID NO: 88) |
| Pae057_phi3 terL_R | reverse | Phi3 | ttgccggacagctcgatatc (SEQ ID NO: 89) |

### Example 5: Characterization of Prophages from Citrobacter freundii and Serratia marcescens by qPCR

Inducible prophages were identified from *Citrobacter freundii* (Cfi) and from *Serratia marcescens* (Sms), two gram-negative bacteria in the Enterobacterales order that have been associated with healthcare associated infections (HAI). In order to determine whether or not these newly identified prophages can be generated into non-replicative transduction particles (NRTPs), qPCR experiments with induced or non-induced cultures were performed, similar to the experiment described in Example 1, with the exception that the primers designed were from tail fiber protein sequences instead of terminase large subunit sequences. The nucleotide sequences, bacteria strain and phage types are shown in TABLE VIII.

**TABLE VIII Primers for prophage characterization in Cfi and Sms strains**

| **Oligonucleotide Name** | **Bacteria strain** | **Phage Type** | **Sequence** |
|---|---|---|---|
| Cfi16709_P1 TF_F forward primer | Cfi | P1 | cggtgcgactgagatcactt (SEQ ID NO: 90) |
| Cfi16709_P1 TF_R reverse primer | Cfi | P1 | actgcgaatgtggatcagct (SEQ ID NO: 91) |
| Cfi16710_phiKO2 TF_F forward primer | Cfi | phiKO2 | cggtatatgaccccacggtg (SEQ ID NO: 92) |
| Cfi16710_phiKO2 TF_R reverse primer | Cfi | phiKO2 | ggttcacggcccggatatag (SEQ ID NO: 93) |
| Sms16017_SEN34 TF_F forward primer | Sms | SEN34 | cgacgagctgcatgttgatg (SEQ ID NO: 94) |
| Sms16017_SEN34 TF_R reverse primer | Sms | SEN34 | ggtcacatctcgaagcgtca (SEQ ID NO: 95) |
| Sms16926_Psp3 TF_F forward primer | Sms | Psp3 | ccttgctggcaaagggtttc (SEQ ID NO: 96) |
| Sms16926_Psp3 TF_R reverse primer | Sms | Psp3 | cagtacccagctccaggttg (SEQ ID NO: 97) |

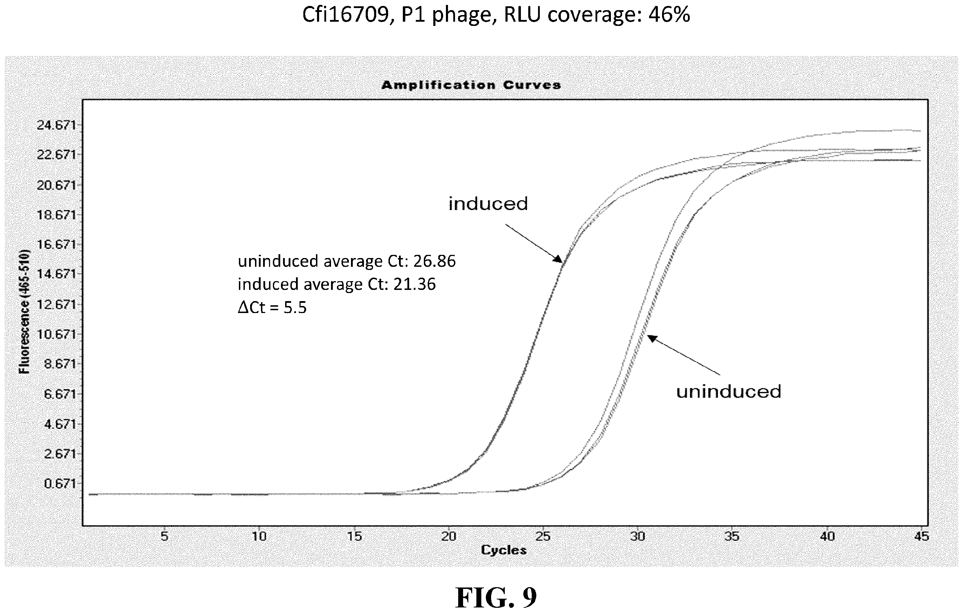

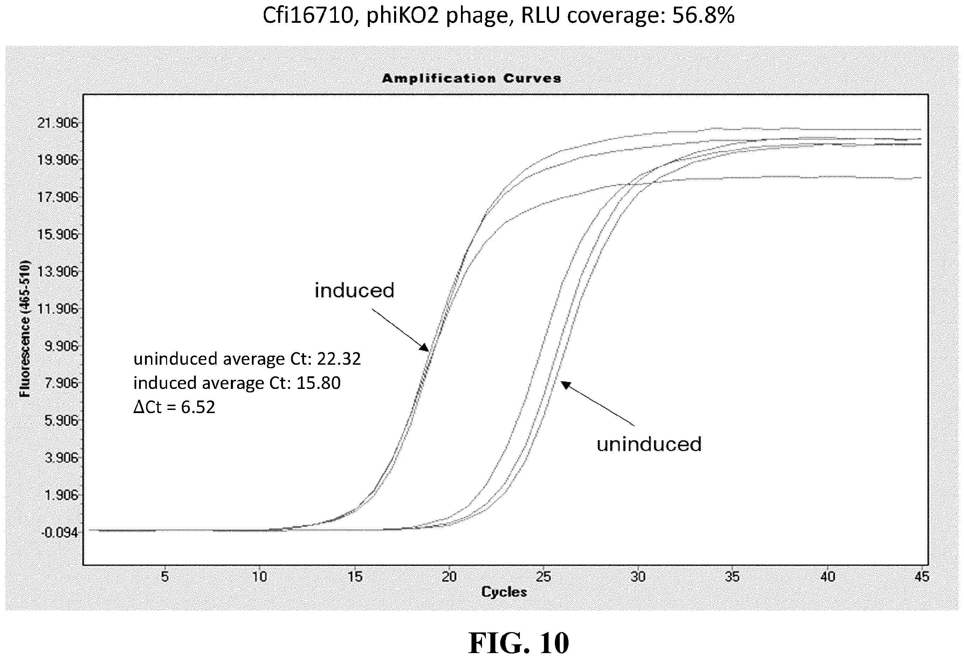

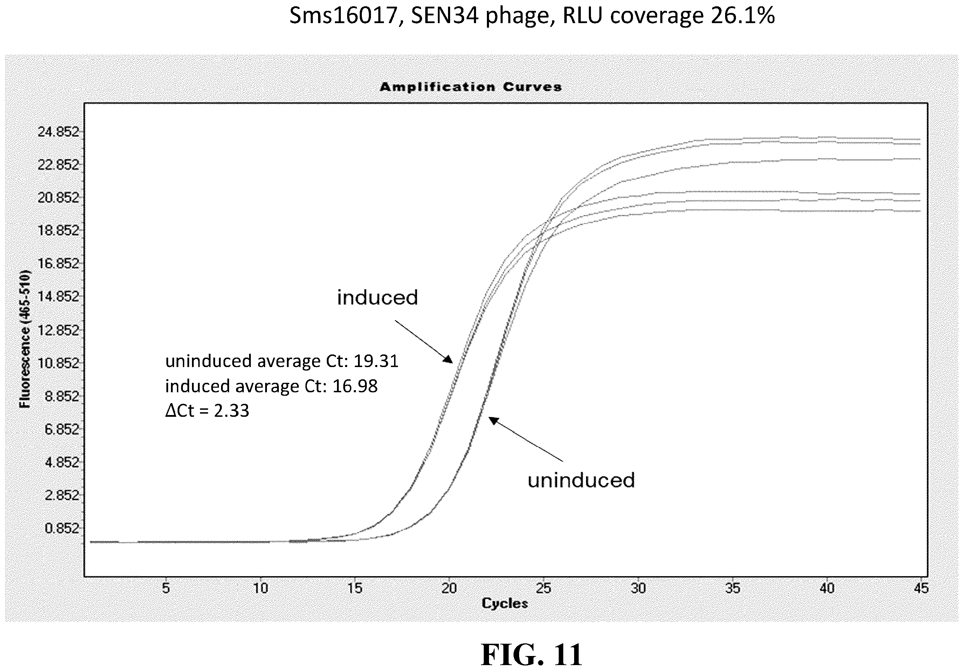

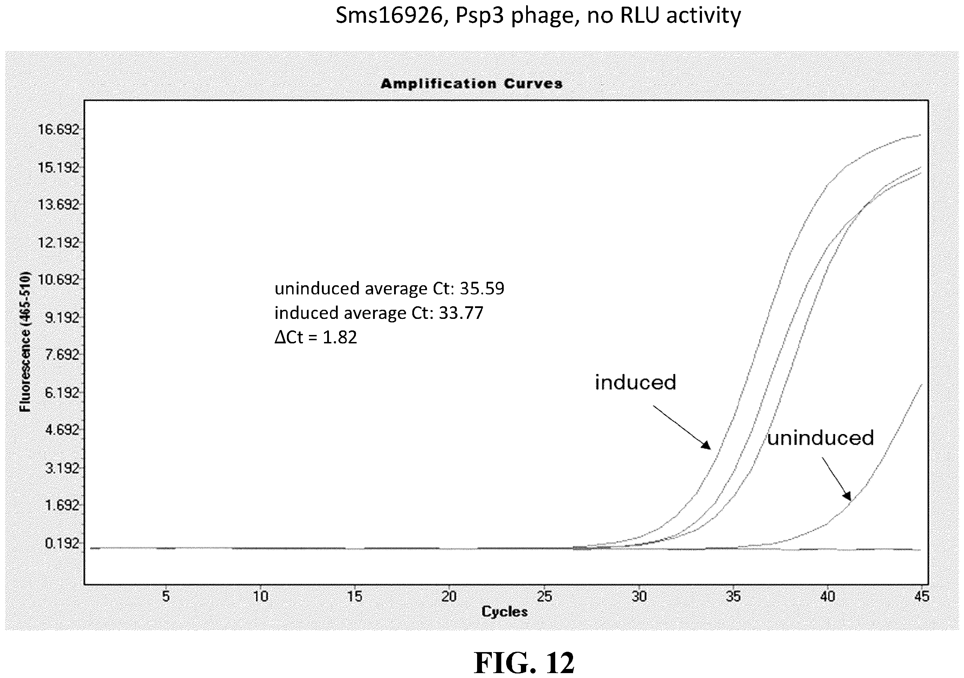

The results of the qPCR experiments are shown as growth curves on FIGs. 9-10 for the Cfi prophages and on FIGs. 11-12 for the Sms prophages. FIGs. 9-10 show that both the P1-type bacteriophage in Cfi strain 16709 and the phiKO2-type bacteriophage in Cfi strain 16710 exhibited ΔCt values of over 5 between induced and non-induced strains and were able to generate functional NRTPs as expressed in light generation from the luciferase gene. In contrast, FIG. 11 shows that while the SEN34-type bacteriophage in Sms strain 16017 which had an induced and non-induced ΔCt of that was slightly over 2 was able to generate functional NRTPs (as shown by light generation), while FIG. 12 shows the Psp3-type bacteriophage in Sms strain 16926 which had a ΔCt that was slightly under 2 between induced and non-induced strains was unable to generate functional NRTPs. In this situation, it is believed that the absolute Ct value of the induced bacteriophage may be important in determining the success of generating functional NRTPs. Although not used in this particular experiment, the demonstration of functional NRTPs can also be performed via a qPCR assay by comparing the growth curves of induced vs non-induced NRTPs using primers that target the luxA gene. Examples of primers that can be used include the following: luxA_qPCR_F forward primer: TGCTGCGGCTAACCTGTTAG (SEQ ID NO: 98), and luxA_qPCR_R reverse primer: TGGTATAGCCCTCGAACGGT (SEQ ID NO: 99).

While the foregoing invention has been described in some detail for purposes of clarity and understanding, it will be clear to one skilled in the art from a reading of this disclosure that various changes in form and detail can be made without departing from the true scope of the invention. For example, all the techniques and apparatus described above can be used in various combinations.

### REFERENCES

Amdt, D., Grant, J., Marcu, A., Sajed, T., Pon, A., Liang, Y., Wishart, D.S. PHASTER: a better, faster version of the PHAST phage search tool. Nucleic Acids Res., 2016 May 3.
Chen, F, Kui Wang, Jeneen Stewart, Robert Belas. Induction of Multiple Prophages from a Marine Bacterium: a Genomic Approach. Appl Environ Microbiol. 2006 Jul; 72(7): 4995-5001. Hertel R, Rodriguez DP, Hollensteiner J, Dietrich S, Leimbach A, Hoppert M, Liesegang H, Volland S.Genome-based identification of active prophage regions by next generation sequencing in Bacillus licheniformis DSM13. PLoS One. 2015 Mar 26;10(3)
Martha RJ Clokie, Andrew D Millard, Andrey V Letarov, Shaun Heaphy. Phages in nature. Bacteriophage. 2011 Jan-Feb; 1(1): 31-45. doi: 10.4161/bact.1.1.14942
Yanlin Zhao,Kui Wang, Hans-Wolfgang Ackermann, Rolf U. Halden, Nianzhi Jiao, and Feng Chen. Searching for a "Hidden" Prophage in a Marine Bacterium. Applied and Environmental Microbiology 2010 Jan, 76 (2)
Zhou,Y., Liang,Y., Lynch,K.H., Dennis,J.J. and Wishart,D. S. PHAST: a fast phage search tool. Nucleic Acids Res., 2011 39, W347-W352.

## Claims

1. A method of identifying the presence of a viable and inducible prophage from a bacterial species that is not known to contain said viable and inducible prophage, said method comprising:
- providing a pair of oligonucleotides to be used as a forward primer and a reverse primer to amplify a segment of a bacteriophage terminase large unit gene;
- performing two quantitative polymerase chain reaction (qPCR) experiments with said forward and reverse primers, wherein one experiment is performed on a culture of said bacterial species that is induced to allow the prophage to undergo the lytic cycle, and the other experiment is performed on a culture of said bacterial species that is not induced;
- comparing the cycle threshold (Ct) values of the two qPCR experiments, wherein a difference of Ct value (ΔCt) between the induced culture and the non-induced culture is greater than two is indicative of the presence of viable and inducible prophage from said bacterial species, and wherein a ΔCt value between the induced culture and the non-induced culture is less than two is indicative of the absence of viable and inducible prophage from said bacterial species.

2. The method of claim 1 further comprising a step of determining the melting temperature (Tm) of the amplified segment, wherein a Tm that is greater than 81°C and less than 89°C is indicative of the presence of viable and inducible prophage from said bacterial species.

3. The method of any one of claims 1 or 2, wherein the nucleotide sequences of the forward primer and the reverse primer are derived from the sequences of bacteriophage terminase large unit genes that have been identified from said bacterial species.

4. The method of any one of claims 1 to 3, wherein the bacterial species is *Klebsiella pneumonia* (Kpn).

5. The method of claim 4 ,wherein the nucleotide sequence of the forward primer is selected from SEQ ID NO: 1-12 and the nucleotide sequence of the reverse primer is selected from SEQ ID NO: 13-24.

6. The method of any one of claims 1 to 3, wherein the bacterial species is *Enterobacter cloacae* (Ecl).

7. The method of claim 6, wherein the nucleotide sequence of the forward primer is selected from SEQ ID NO: 25-34 and the nucleotide sequence of the reverse primer is selected from SEQ ID NO: 35-44.

8. The method of any one of claims 1 to 3, wherein the bacterial species is *Escherichia coli* (Eco).

9. The method of claim 8, wherein the nucleotide sequence of the forward primer is selected from SEQ ID NO: 45-62 and the nucleotide sequence of the reverse primer is selected from SEQ ID NO: 63-80.

10. The method of any one of claims 1 to 3, wherein the bacterial species is *Pseudomonas aeruginosa* (Pae).

11. The method of claim 10, wherein the nucleotide sequence of the forward primer is selected from SEQ ID NOs: 82, 84, 86 or 88 and the nucleotide sequence of the reverse primer is selected from SEQ ID NOs: 83, 85, 87 or 89.

12. A packaging plasmid comprising a terminase small unit gene and a terminase large unit gene derived from an induced prophage, wherein the induced prophage is derived from *Klebsiella pneumonia* (Kpn) and comprises a nucleotide sequence of pZX023 (SEQ ID NO: 81).

13. A non-replicative transduction particle that comprises the packaging plasmid of claim 12.

14. A method of identifying an inducible prophage from a bacteria species for creation of a functional non-replicative transcription particle (NRTP) that is **characterized by** delivering into a cell a reporter molecule comprising a detectable reporter gene and expressing the detectable reporter gene in the cell, said method comprising:
- providing a pair of oligonucleotides to be used as a forward primer and a reverse primer to amplify a segment of a bacteriophage terminase large unit gene or a bacteriophage tail fiber protein gene;
- performing two quantitative polymerase chain reaction (qPCR) experiments with said forward and reverse primers, wherein one experiment is performed on a culture of said bacterial species that is induced to allow the prophage to undergo the lytic cycle, and the other experiment is performed on a culture of said bacterial species that is not induced;
- comparing the cycle threshold (Ct) values of the two qPCR experiments, wherein a difference of Ct value (ΔCt) between the induced culture and the non-induced culture is greater than two is indicative of the ability of the bacteriophage to generate the functional NRTP, and wherein a ΔCt value between the induced culture and the non-induced culture is less than two is indicative of the inability of the bacteriophage to generate the functional NRTP.

15. The method of claim 14, wherein the bacteria species is from the Enterobacteriales order.

16. The method of claim 15, wherein the bacteria species is *Citrobacter freundii* (Cfi) or wherein the bacteria species is *Serratia marcescens* (Sms).

## Patentansprüche

1. Verfahren zum Identifizieren der Gegenwart eines lebensfähigen und induzierbaren Prophagen aus einer Bakterienspezies, von der nicht bekannt ist, ob sie den lebensfähigen und induzierbaren Prophagen enthält, wobei das Verfahren Folgendes umfasst:
- Bereitstellen eines Paares von Oligonukleotiden zur Verwendung als ein Vorwärts-Primer und ein Rückwärts-Primer zum Amplifizieren eines Segments eines Bakteriophagen-Gens der großen Terminase-Einheit;
- Durchführen von zwei quantitativen Polymerasekettenreaktion-(qPCR)-Experimenten mit dem Vorwärts- und Rückwärts-Primer, wobei ein Experiment an einer Kultur von der Bakterienspezies durchgeführt wird, die induziert wird, um dem Prophagen zu ermöglichen, den lytischen Zyklus zu durchlaufen, und das andere Experiment an einer Kultur von der Bakterienspezies durchgeführt wird, die nicht induziert wird;
- Vergleichen der Zyklusschwellen-(Ct)-Werte der beiden qPCR-Experimente, wobei eine Differenz des Ct-Wertes (ΔCt) zwischen der induzierten Kultur und der nicht-induzierten Kultur größer als zwei die Gegenwart eines lebensfähigen und induzierbaren Prophagen aus der Bakterienspezies angibt und wobei ein ΔCt-Wert zwischen der induzierten Kultur und der nicht-induzierten Kultur kleiner als zwei die Abwesenheit eines lebensfähigen und induzierbaren Prophagen aus der Bakterienspezies angibt.

2. Verfahren nach Anspruch 1, ferner umfassend einen Schritt des Bestimmens der Schmelztemperatur (Tm) des amplifizierten Segments, wobei eine Tm, die höher ist als 81 °C und niedriger als 89 °C, die Gegenwart eines lebensfähigen und induzierbaren Prophagen aus der Bakterienspezies angibt.

3. Verfahren nach einem der Ansprüche 1 oder 2, wobei die Nukleotidsequenzen des Vorwärts-Primers und des Rückwärts-Primers von den Sequenzen von Bakteriophagen-Genen der großen Terminase-Einheit abgeleitet sind, die aus der Bakterienspezies identifiziert worden sind.

4. Verfahren nach einem der Ansprüche 1 bis 3, wobei die Bakterienspezies *Klebsiella pneumonia* (Kpn) ist.

5. Verfahren nach Anspruch 4, wobei die Nukleotidsequenz des Vorwärts-Primers aus SEQ ID NO:1-12 ausgewählt ist und die Nukleotidsequenz des Rückwärts-Primers aus SEQ ID NO:13-24 ausgewählt ist.

6. Verfahren nach einem der Ansprüche 1 bis 3, wobei die Bakterienspezies *Enterobacter cloacae* (Ecl) ist.

7. Verfahren nach Anspruch 6, wobei die Nukleotidsequenz des Vorwärts-Primers aus SEQ ID NO:25-34 ausgewählt ist und die Nukleotidsequenz des Rückwärts-Primers aus SEQ ID NO:35-44 ausgewählt ist.

8. Verfahren nach einem der Ansprüche 1 bis 3, wobei die Bakterienspezies *Escherichia coli* (Eco) ist.

9. Verfahren nach Anspruch 8, wobei die Nukleotidsequenz des Vorwärts-Primers aus SEQ ID NO:45-62 ausgewählt ist und die Nukleotidsequenz des Rückwärts-Primers aus SEQ ID NO:63-80 ausgewählt ist.

10. Verfahren nach einem der Ansprüche 1 bis 3, wobei die Bakterienspezies *Pseudomonas aeruginosa* (Pae) ist.

11. Verfahren nach Anspruch 10, wobei die Nukleotidsequenz des Vorwärts-Primers aus SEQ ID NO:82, 84, 86 oder 88 ausgewählt ist und die Nukleotidsequenz des Rückwärts-Primers aus SEQ ID NO:83, 85, 87 oder 89 ausgewählt ist.

12. Verpackungsplasmid, umfassend ein Gen der kleinen Terminase-Einheit und ein Gen der großen Terminase-Einheit, abgeleitet von einem induzierten Prophagen, wobei der induzierte Prophage von *Klebsiella pneumonia* (Kpn) abgeleitet ist und eine Nukleotidsequenz von pZX023 (SEQ ID NO:81) umfasst.

13. Nicht-replikatives Transduktionspartikel, welches das Verpackungsplasmid nach Anspruch 12 umfasst.

14. Verfahren zum Identifizieren eines induzierbaren Prophagen aus einer Bakterienspezies zur Erzeugung eines funktionellen nicht-replikativen Transkriptionspartikels (NRTP), das **dadurch gekennzeichnet ist, dass** es ein Reportermolekül in eine Zelle abgibt, das ein nachweisbares Reportergen umfasst und das nachweisbare Reportergen in der Zelle exprimiert, wobei das Verfahren Folgendes umfasst:
- Bereitstellen eines Paares von Oligonukleotiden zur Verwendung als ein Vorwärts-Primer und ein Rückwärts-Primer zum Amplifizieren eines Segments eines Bakteriophagen-Gens der großen Terminase-Einheit oder eines Bakteriophagen-Gens des Schwanzfaserproteins;
- Durchführen von zwei quantitativen Polymerasekettenreaktion-(qPCR)-Experimenten mit dem Vorwärts- und Rückwärts-Primer, wobei ein Experiment an einer Kultur von der Bakterienspezies durchgeführt wird, die induziert wird, um dem Prophagen zu ermöglichen, den lytischen Zyklus zu durchlaufen, und das andere Experiment an einer Kultur von der Bakterienspezies durchgeführt wird, die nicht induziert wird;
- Vergleichen der Zyklusschwellen-(Ct)-Werte der beiden qPCR-Experimente, wobei eine Differenz des Ct-Wertes (ΔCt) zwischen der induzierten Kultur und der nicht-induzierten Kultur größer als zwei die Fähigkeit des Bakteriophagen, das funktionelle NRTP zu erzeugen, angibt, und wobei ein ΔCt-Wert zwischen der induzierten Kultur und der nicht-induzierten Kultur kleiner als zwei die Unfähigkeit des Bakteriophagen, das funktionelle NRTP zu erzeugen, angibt.

15. Verfahren nach Anspruch 14, wobei die Bakterienspezies aus der Ordnung der Enterobakterien stammt.

16. Verfahren nach Anspruch 15, wobei die Bakterienspezies *Citrobacter freundii* (Cfi) ist oder wobei die Bakterienspezies *Serratia marcescens* (Sms) ist.

## Revendications

1. Procédé d'identification de la présence d'un prophage viable et inductible à partir d'une espèce bactérienne qui n'est pas connue pour contenir ledit prophage viable et inductible, ledit procédé comprenant :
- la fourniture d'une paire d'oligonucléotides à utiliser comme amorce sens et amorce antisens pour amplifier un segment d'un gène de grosse unité de terminase de bactériophage ;
- la réalisation de deux expériences de réaction d'amplification en chaîne par polymérase quantitative (qPCR) avec lesdites amorces sens et antisens, dans lequel une expérience est réalisée sur une culture de ladite espèce bactérienne qui est induite pour permettre au prophage de subir le cycle lytique, et l'autre expérience est réalisée sur une culture de ladite espèce bactérienne qui n'est pas induite ;
- la comparaison des valeurs de cycle seuil (Ct) des deux expériences qPCR, dans lequel une différence de valeurs Ct (ΔCt) entre la culture induite et la culture non induite est supérieure à deux indique la présence d'un prophage viable et inductible à partir de ladite espèce bactérienne, et dans lequel une valeur ΔCt entre la culture induite et la culture non induite est inférieure à deux indique l'absence d'un prophage viable et inductible à partir de ladite espèce bactérienne.

2. Procédé selon la revendication 1 comprenant en outre une étape de détermination de la température de fusion (Tm) du segment amplifié, dans lequel une Tm qui est supérieure à 81 °C et inférieure à 89 °C indique la présence d'un prophage viable et inductible à partir de ladite espèce bactérienne.

3. Procédé selon l'une quelconque des revendications 1 ou 2, dans lequel les séquences nucléotidiques de l'amorce sens et de l'amorce antisens sont dérivées des séquences de gènes de grosse unité de terminase de bactériophage qui ont été identifiées à partir de ladite espèce bactérienne.

4. Procédé selon l'une quelconque des revendications 1 à 3, dans lequel l'espèce bactérienne est *Klebsiellapneumonia* (Kpn).

5. Procédé selon la revendication 4, dans lequel la séquence nucléotidique de l'amorce sens est choisie parmi les SEQ ID NO : 1 à 12 et la séquence nucléotidique de l'amorce antisens est choisie parmi les SEQ ID NO : 13 à 24.

6. Procédé selon l'une quelconque des revendications 1 à 3, dans lequel l'espèce bactérienne est *Enterobacter cloacae* (Ecl).

7. Procédé selon la revendication 6, dans lequel la séquence nucléotidique de l'amorce sens est choisie parmi les SEQ ID NO : 25 à 34 et la séquence nucléotidique de l'amorce antisens est choisie parmi les SEQ ID NO : 35 à 44.

8. Procédé selon l'une quelconque des revendications 1 à 3, dans lequel l'espèce bactérienne est *Escherichia coli* (Eco).

9. Procédé selon la revendication 8, dans lequel la séquence nucléotidique de l'amorce sens est choisie parmi les SEQ ID NO : 45 à 62 et la séquence nucléotidique de l'amorce antisens est choisie parmi les SEQ ID NO : 63 à 80.

10. Procédé selon l'une quelconque des revendications 1 à 3, dans lequel l'espèce bactérienne est *Pseudomonas aeruginosa* (Pae).

11. Procédé selon la revendication 10, dans lequel la séquence nucléotidique de l'amorce sens est choisie parmi les SEQ ID NO : 82, 84, 86 ou 88 et la séquence nucléotidique de l'amorce antisens est choisie parmi les SEQ ID NO : 83, 85, 87 ou 89.

12. Plasmide d'encapsidation comprenant un gène de petite unité de terminase et un gène de grosse unité de terminase dérivés d'un prophage induit, dans lequel le prophage induit est dérivé de *Klebsiella pneumonia* (Kpn) et comprend une séquence nucléotidique de pZX023 (SEQ ID NO : 81).

13. Particule transductrice non réplicative qui comprend le plasmide d'encapsidation selon la revendication 12.

14. Procédé d'identification d'un prophage inductible à partir d'une espèce bactérienne pour la création d'une particule transductrice non réplicative (NRTP) fonctionnelle qui est **caractérisé par** l'administration dans une cellule d'une molécule rapporteur comprenant un gène rapporteur détectable et l'expression du gène rapporteur détectable dans la cellule, ledit procédé comprenant :
- la fourniture d'une paire d'oligonucléotides à utiliser comme amorce sens et amorce antisens pour amplifier un segment d'un gène de grosse unité de terminase de bactériophage ou d'un gène de protéine à fibre caudale de bactériophage ;
- la réalisation de deux expériences de réaction d'amplification en chaîne par polymérase quantitative (qPCR) avec lesdites amorces sens et antisens, dans lequel une expérience est réalisée sur une culture de ladite espèce bactérienne qui est induite pour permettre au prophage de subir le cycle lytique, et l'autre expérience est réalisée sur une culture de ladite espèce bactérienne qui n'est pas induite ;
- la comparaison des valeurs de cycle seuil (Ct) des deux expériences qPCR, dans lequel une différence de valeurs Ct (ΔCt) entre la culture induite et la culture non induite est supérieure à deux indique la capacité du bactériophage à générer la NRTP fonctionnelle, et dans lequel une valeur ΔCt entre la culture induite et la culture non induite est inférieure à deux indique l'incapacité du bactériophage à générer la NRTP fonctionnelle.

15. Procédé selon la revendication 14, dans lequel l'espère bactérienne fait partie de l'ordre des Enterobacterales.

16. Procédé selon la revendication 15, dans lequel l'espèce bactérienne est *Citrobacter freundii* (Cfi) ou dans lequel l'espèce bactérienne est *Serratia marcescens* (Sms).
